# EUROPEAN PATENT SPECIFICATION

(11) **EP 3 082 769 B1**
(45) Date of publication and mention of the grant of the patent: **31.01.2018**
(21) Application number: 14815714.2
(22) Date of filing: 18.12.2014
(51) Int. Cl.: A61K 9/133, A61K 39/145, A61K 39/12, A61K 9/127, A61K 39/00, A61K 9/00, A61K 9/51, A61K 47/02, A61K 47/26

(54) **IMPROVED FORMULATIONS FOR VIROSOMES**
VERBESSERTE FORMULIERUNGEN FÜR VIROSOME
FORMULATIONS AMÉLIORÉES POUR DES VIROSOMES

(30) Priority: 19.12.2013 EP 13198296
(43) Date of publication of application: 26.10.2016
(73) Proprietor: Janssen Vaccines & Prevention B.V., 2333 CN Leiden (NL)
(72) Inventor: ADRIAANSEN, Janik, 2333 CN Leiden (NL); DORO, Francesco, 2333 CN Leiden (NL)
(74) Representative: Beslier, Victor
(86) International application number: PCT/EP2014/078463
(87) International publication number: WO 2015/091798

(56) References cited:
- EP-A1- 1 676 569
- WO-A1-2004/045582
- WO-A2-2011/090712
- JP-A- S6 393 727
- US-A1- 2004 028 687
- ANDREAS R. KAMMER ET AL: "A new and versatile virosomal antigen delivery system to induce cellular and humoral immune responses", VACCINE, vol. 25, no. 41, 1 October 2007 (2007-10-01), pages 7065-7074, XP055109819, ISSN: 0264-410X, DOI: 10.1016/j.vaccine.2007.07.052
- MISCHLER R ET AL: "Inflexal<(>R)V a trivalent virosome subunit influenza vaccine: production", VACCINE, ELSEVIER LTD, GB, vol. 20, 20 December 2002 (2002-12-20), pages B17-B23, XP004397481, ISSN: 0264-410X, DOI: 10.1016/S0264-410X(02)00512-1
- PAULA FREIXEIRO ET AL: "Study of the stability of proteoliposomes as vehicles for vaccines against Neisseria meningitidis based on recombinant porin complexes", INTERNATIONAL JOURNAL OF PHARMACEUTICS, vol. 443, no. 1-2, 1 February 2013 (2013-02-01), pages 1-8, XP055108675, ISSN: 0378-5173, DOI: 10.1016/j.ijpharm.2012.12.046
- HINCHA DIRK K ET AL: "Trehalose increases freeze-thaw damage in liposomes containing chloroplast glycolipids", CRYOBIOLOGY, vol. 36, no. 3, May 1998 (1998-05), pages 245-249, XP002722282, ISSN: 0011-2240
- WILSCHUT JAN ET AL: "Preservation of influenza virosome structure and function during freeze-drying and storage.", JOURNAL OF LIPOSOME RESEARCH 2007, vol. 17, no. 3-4, 2007, pages 173-182, XP009177150, ISSN: 0898-2104

## Description

### FIELD OF THE INVENTION

The present invention relates to formulations for virosomes and related pharmaceutical products for use in therapeutic and vaccine applications. In particular, liquid formulations for virosomes are disclosed herein, which widen the temperature window for the virosomes to remain stable. This is done by preserving virosome quantity and physical and chemical properties, as well as proteinaceous components and structural integrity when stored in about the 2-8°C range or higher while also preventing damage deriving from accidental freezing and being compatible with parenteral administration.

### BACKGROUND OF THE INVENTION

A virosome is a vesicle comprising a unilamellar phospholipid membrane incorporating virus derived proteins, this combination allows the virosome to fuse with target cells. Virosomes are consequently different from liposomes and considered to be very efficient drug or vaccine delivery systems.

An ongoing challenge in the field of drug delivery and vaccine research is to generate liquid formulations for virosomes, wherein the virosomes remain stable over a long period and within a wide temperature window. In fact, any accidental freezing (e.g. during storage or transportation) has a detrimental impact on product integrity and therefore efficacy. Any accidental heating is also detrimental, causing product instability and efficacy loss (e.g. due to aggregation). A longer stability within a wide storage temperature range, such as from about 2°C to about 8°C, leading to a longer shelf life, is generally desirable for any injectable liquid formulation.

The biological activity of a virosome particle depends upon the conformational integrity of the particle and upon the quality of the antigenic molecules that are associated with its membrane. Unlike traditional organic and inorganic drugs, virosomal particles are complex and built from many specific phospholipids and proteins where minor chemical or physical stressors can contribute to the degradation of the virosomal particle. A stable composition for a virosomal preparation is therefore of crucial importance to ensure a long shelf-life, but stabilizing virosomes poses particular challenges. Virosomes may lose potency as a result of physical instabilities, including denaturation of the embedded proteins, particle aggregation (both soluble and insoluble aggregate formation) or fusion, dissociation, precipitation and adsorption as well as chemical instabilities, including, for example, hydrolysis, deamidation, and oxidation. Furthermore, lipids which are structural components of the virosomes, are susceptible to hydrolysis and oxidation. Any of these degradation routes can lead to lowered biologically activity, but can potentially also result in the formation of by-products or derivatives having increased toxicity, and/or altered immunogenicity.

It therefore needs a tailored approach to find a robust composition for virosomes ensuring stability over a wide range of conditions. Buffer type, pH and specialized excipients will need to be combined in unique combinations and meticulously optimized to keep a virosome chemically, physically and biologically stable. In view of all the factors that can be varied, finding optimal conditions for formulating virosomes is burdened with challenges, and the composition of a good composition is *a priori* unpredictable.

Formulations comprising liposomes have been disclosed previously. Although liposomes are fundamentally different from virosomes, these formulations could be considered as related prior art. Many lyophilized liposome formulations exist on the market, like AmBisome®, (Gilead Sciences, Inc, San Dimas, CA), Amphotec® (Ben Venue Laboratories, Inc, Bedford, OH), Myocet, Visudyne® (Novartis Pharma AG, Basel, Switzerland), and LEP-ETU (liposome-entrapped paclitaxel easy-to-use; NeoPharm, Inc, Lake Bluff, IL; Freixeiro et al.; Meunier et al.) and they are reasonably stable, however they are expensive and require time consuming handling, prone to error, before administration. A liquid composition that can be stored between 2-8°C or ≤-65°C would therefore be a preferred product form for virosomes. In particular if said composition would allow the virosomes to be resistant to accidental freezing during transportation or storage itself.

In the prior art, only a few examples studying the use of mono or disaccharides in liposome liquid formulations have been described. Interestingly, the effect of such mono or disaccharides are found to be detrimental, as they lead to a dramatic increase in freeze-thaw damage (Hincha et al) or decrease the stability during storage at 2-8°C (Freixeiro et al).

Liquid formulations for virosomes have been disclosed previously and have been used for many years in marketed products, such as Inflexal®V and Epaxal®. Said formulations were shown herein to be suboptimal, in the sense that they are not able to preserve the stability of virosomes after having been frozen. Despite the fact that these formulations have been on the market for many years, manufacturing companies have not managed to make virosomal formulations resistant to an accidental freezing event or long term low temperature storage (i.e. ≤ -65°C).

The identification of a formulation capable of preventing damage to virosome structure and antigen content from accidental freezing, while ensuring optimal stability during storage at 2-8°C remains a challenge, and would lead to the huge advantage of reducing costs and facilitating the clinical application of said formulation.

Accordingly, there is a need in the art to find liquid formulations that preserve the stability of virosomes when accidentally frozen during storage or transportation. These improved formulations will preserve the quantity and quality of the contained virosomes during storage over a prolonged period of time. Furthermore, the formulation should be suitable for parenteral administration, should be well tolerated and should preferably have a simple composition. It is an object of the invention to provide such formulations for virosomes.

### SUMMARY OF THE INVENTION

We have found and describe herein, compositions that preserve the stability of virosomes when accidentally frozen, thereby improving the virosomal stability by preserving quantity and quality of the virosomes as compared to previously disclosed compositions (or formulations). Surprisingly, the combination of a combined KH₂PO₄/Na₂HPO₄ buffer having a pH ranging between 6.5 and 8 together with trehalose resulted in an outstanding composition for the preservation of quantity and quality of virosomes after having been frozen, therewith improving overall virosomal stability as compared to other compositions known in the art.

The present invention therefore relates to a liquid composition (or formulation) for stabilizing virosomes and related pharmaceutical products that can e.g. be used in therapeutic and vaccine applications. The liquid compositions according to the present invention comprise a) a virosome in a b) combined KH₂PO₄/Na₂HPO₄ buffer at a pH ranging between 6.5 and 8, wherein the phosphate concentration is ranging between 15 mM and 30 mM c) a salt at a concentration higher than 60 mM; and further comprise d) trehalose, wherein the trehalose concentration is ranging between 2% w/w an 10% w/w. The virosomal formulations of the present invention are amenable to prolonged storage at 2°C to 8°C or ≤-65°C, for more than 6 months, 1 year, 1.5 year, 2 years or more. Preferably the composition according to the present invention comprises virosomes with a total antigen concentration between about 20 µg/mL and 300 µg/mL. In a preferred embodiment according to the present invention the composition has a pH ranging between 7 and 7.8.

In a preferred embodiment according to the present invention the composition has a pH ranging between 7 and 7.8; comprises a KH₂PO₄/Na₂HPO₄ buffer wherein the phosphate concentration is ranging between 15 mM and 30 mM; the composition also comprises a salt at a concentration higher than 60 mM; and further comprises trehalose at a concentration ranging between 2% w/w and 10% w/w. In yet another preferred embodiment according to the present invention the composition has a pH of 7.5; comprises a KH₂PO₄/Na₂HPO₄ buffer wherein the phosphate concentration is ranging between 15 mM and 30 mM; the composition also comprises a salt at a concentration higher than 60 mM; and further comprises trehalose at a concentration ranging between 3% w/w and 5% w/w. In a preferred embodiment the composition according to the present invention comprises a salt at a concentration ranging between 60 and 85 mM. In another preferred embodiment said salt is NaCl. In one embodiment, the compositions according to the present invention are contained in a vial. In another embodiment, the compositions are contained in a bag. In yet another embodiment the compositions are contained in a (pre-filled) syringe or cartridge.

The present invention also relates to a method of preserving a virosome which comprises preparing a composition according to any one of claims 1-5. In yet another embodiment, the present invention relates to a method of preserving a virosome which comprises preparing a composition according to any of claims 1-5 and storing said composition at a temperature ranging between 2°C and 8°C. In certain embodiments, said composition is stored for more than 6 months, 1 year, 1.5 year, 2 years or more.

In other embodiments, the present invention relates to a method of preserving a virosome which comprises preparing a composition according to any of claims 1-5 and storing said composition at a temperature ranging between -15°C and -30°C.

In other embodiments, the present invention relates to a method of preserving a virosome which comprises preparing a composition according to any of claims 1-5 and storing said composition at a temperature ranging between -80°C and -65°C.

The enhanced long-term stability over a wide temperature range results in an extended shelf life of the virosome compositions (or formulations) disclosed herein, allowing for storage and eventual host administration of these compositions over about a 1-2 year period with acceptable losses in active monovalent antigen concentration (i.e. not more than 27% loss in terms of HA concentration, at 2-8°C). In addition, compositions of the present invention show stability during exposure to elevated temperatures, freeze/thaw cycles or long term low temperature storage (i.e. ≤ -65°C).

### DESCRIPTION OF THE FIGURES

Figure 1 (A and B). Average diameter size and HA concentration have been measured for Influenza(A/California) derived virosomes before and after one freeze/thaw cycle.
Figure 2 (A and B). Influenza (A/California) derived virosomes were stored at 5±3°C for 12 weeks. Average diameter size and HA concentration were measured at t=0, t= 4 and t=12 weeks.
Figure 3 (A and B). Average diameter size and HA concentration have been measured for Influenza (B/Brisbane) derived virosomes before and after one freeze/thaw cycle.
Figure 4 (A and B). Influenza (B/Brisbane) derived virosomes were stored at 5±3°C for 12 weeks. Average diameter size was measured at t=0, t= 4, t=12 and t=24 weeks and HA concentration was measured at t=0 t= 4 and t= 12
Figure 5 (A and B). Average diameter size and HA concentration were measured for Influenza (A/Victoria) derived virosomes before and after one freeze/thaw cycle.
Figure 6 (A and B). Influenza (A/Victoria) derived virosomes were stored at 5±3°C for 12 weeks. Average diameter size was measured at t=0 and t= 4 weeks and HA concentration was measured at t=0, t= 4 and t=12 weeks.
Figures 7 (A, B and C). Temperature dependent profile obtained by measuring virosome size variation over temperature to identify aggregation onset temperature and overall size variation of virosomes derived from three different Influenza strains, in the different formulations tested.
Figure 8 Titration profile measuring cell derived A/Victoria virosome size variation over salt concentration to identify aggregation onset and overall size variation upon salt concentration decrease.
Figure 9 (A and B). Average diameter size and HA concentration were measured for cell cultured A/California derived virosomes before and after one freeze/thaw cycle.
Figure 10 (A and B). Cell cultured A/California derived virosomes were stored at 5±3°C for 12 weeks and average diameter size and HA concentration were measured at t=0, t= 4 and t=12 weeks.
Figure 11 (A and B). Average diameter size and HA concentration were measured for cell cultured B/Brisbane derived virosomes before and after one freeze/thaw cycle.
Figure 12 (A and B). Cell cultured B/Brirbane derived virosomes were stored at 5±3°C for 4 weeks and average diameter size and HA concentration were measured at t=0, t= 4 ant t=12 weeks.
Figure 13 (A and B). Average diameter size and HA concentration were measured for A/Victoria derived virosomes before and after one freeze/thaw cycle.
Figure 14 (A and B). A/Victoria derived virosomes were stored at 5±3°C for 12 weeks and average diameter size and HA concentration were measured at t=0, t= 4 ant t=12 weeks.
Figure 15 (A, B, C and D). Average diameter size and HA concentration were measured for blended trivalent virosomes before and after one freeze/thaw cycle.
Figure 16 (A, B, C and D). Trivalent blended virosomes were stored at 5±3°C for 12 weeks and average diameter size and HA concentration were measured at t=0, t= 4 ant t=12 weeks.
Figure 17 (A, B, C and D). Trivalent blended virosomes were stored at <-65°C for 12 weeks and average diameter size and HA concentration were measured at t=0 and t=12 weeks.

### DETAILED DESCRIPTION OF THE INVENTION

The formulations of the invention comprise at least one virosome. A virosome is a drug or vaccine delivery mechanism consisting of unilamellar phospholipid membrane vesicle incorporating virus derived proteins to allow the virosomes to fuse with target cells. Virosomes are not able to replicate but are pure fusion-active vesicles.

Virosomes are reconstituted phospholipid (PL) membranes containing proteins from a virus, like hemagglutinin (HA) and neuraminidase (NA) from an influenza virus. The HA and NA antigens are preferably originating from an influenza strain, such as but not limiting to an A/California strain, B/Brisbane strain or A/Victoria strain. In a preferred embodiment the influenza virus strains used in the present invention are selected from the group of A/California strain, B/Brisbane and A/Victoria.

The virosomes in the compositions (or formulations) of the present invention may be derived from influenza viruses, or from other enveloped viruses, such as, but not limited to the following families: flaviviridae (e.g. Dengue virus, Hepatitis C virus HEV, Japanese encephalitis virus, Yellow fever virus, West Nile virus), Poxviridae (i.e. Cowpox virus, Monkeypox virus, vaccinia virus, Variola virus), Retroviridae (i.e. Immuno deficiency viruses HIV/SIV, paramyxoviridae (i.e. Measles virus, Mumps virus, Parainfluenza viruses, metapneumovirus, Respiratory Syncytial virus RSV), and Orthomyxoviridae (i.e. influenza viruses). Since virosomes do not contain the viral genomic material (e.g. viral RNA or DNA), they are non-replicative by nature, which renders them safe for administration to animals and humans in the form of an immunogenic composition (e.g. as a vaccine), or as an adjuvant, or as drug (protein) delivery vesicle with or without targeting ligands. Virosomes thus have been especially useful in the field of vaccination, where it is desired to stimulate an immune response to an antigen or antigens associated with a particular disease or disorder. In such cases, an antigen (or antigens) is typically encapsulated or embedded in the virosome or associated with virosome, which then delivers this antigen or the antigens to the host immune system of the subject to be vaccinated.

Virosomes therefore represent an innovative, broadly applicable carrier system having adjuvant properties with prospective applications in areas beyond conventional vaccines. Virosomes are considered to be very efficient and widely used drug or vaccine delivery systems.

Virosomes are generally produced from a solubilized virus fraction using either of two different approaches, one approach involving the addition of exogenous lipids to the solubilized virus fraction (as described in e.g.US2009/0263470, US2009/0087453) prior to reconstitution of the virosomal membranes, and the other approach being based on reconstituting the viral membrane without the addition of exogenous lipids (e.g. as described in US 7,901,920). The construction of virosomes is well understood in the art and involves the use of standard techniques, such as those described in, for example, Stegmann et al., Mishler et al. and Herzog et al. The mode of administration can be, but is not limited to, intramuscular, intra-dermal and intra-nasal.

The term "stability" as used herein refers to the tendency of a virosome particle to resist to degradation in a formulation, thereby retaining its biological effect on the timescale of its expected usefulness.

A virosome "retains its physical stability" in a composition or pharmaceutical formulation, if it, amongst others, shows minimal loss in terms of quantity (i.e. not more than 27% loss in terms of HA concentration) and biological activity, and displays no major protein modifications. Additionally, no signs of aggregation, dissociation, precipitation, changing of colour and/or clarity upon visual examination should be observed.

"About" as used in the present application means ±10%, unless stated otherwise.

By "pharmaceutically acceptable excipient" is meant any inert substance that is combined with an active molecule such as a virosome for preparing an agreeable or convenient dosage form. The "pharmaceutically acceptable excipient" is an excipient that is non-toxic to recipients at the dosages and concentrations used; and is compatible with other ingredients of the composition comprising the virosomal preparation. Examples of pharmaceutical acceptable excipients are cryoprotectants, non-ionic detergents, buffers, salts, inhibitors of free radical oxidation approved by the Food and Drug Administration (FDA).

The term "by-product" includes undesired products, which detract or diminish the proportion of active virosomes in a given formulation. Typical by-products include virosome aggregates. These are soluble or insoluble complexes that have a particle size greater than the virosomes. In addition to virosomes aggregates, virosome degradation products may include, for example, unstructured virosomes, protein aggregates or precipitated material.

A composition (interchangeably named formulation) which improves the virosomal stability, also named a "stable formulation" as used herein is a composition in which the virosomes therein essentially retain their physical and/or chemical integrity and/or biological activity upon storage. Stability can be assessed by determining different characteristics such as the concentration of the protein(s) contained in the virosome formulation, the lipid content, the potency, and/or other quality aspects of the virosomes in the composition over a period of time and under certain storage conditions. In particular the average particle size may be measured as indication of the aggregation state of the virosomes and should be between about 50 and 300 nm, ideally between 100 and 250 nm. The characteristics of a virosome composition can be measured at elevated temperatures or under other stressed conditions, for instance formulations can be subjected to incubation at 25°C or subjected to freeze/thaw cycles and agitation in order to study the effects of different formulations on the shelf-life. Said characteristics which determine the stability may be determined by at least one of the methods selected from the group consisting of visual inspection, the Single Radial ImmunoDiffusion (SRID) method and Zeta sizer measurements or other applicable methods

### Single radial immunodiffusion method (SRID)

The single radial immunodiffusion method, as described in Wood et al. 1977, leads to the quantitative and qualitative determination of HA in virosomal samples. The antigens are solubilized in a detergent (Zwittergent 3-14 detergent, VWR) to allow their diffusion in an agar gel containing strain specific antibodies. Antigen and antibodies will bind and form small and soluble antigen-antibody complexes when the antigen is present in an excess. Due to the diffusion into the gel, a larger number of antibodies will bind until the equilibrium is reached and an insoluble precipitation ring is formed. The quantity of the insoluble antigen-antibody complex at the external edge of the circle increases over time and therefore the diameter of the circle increases over time. The squared diameter (and the area) of the circle is directly proportional to the initial concentration of the antigen and inversely proportional to the antibody concentration in the gel. Measuring the diameter size and comparing it with a standard curve, will give the concentration of the active antigen in the sample.

As an internal control, inactivated egg-derived influenza virus of the appropriate strains have been be used. Each internal control was calibrated against the international standard.

Precipitation rings diameters (in mm) are measured using the Immulab software or alternatively they can be measured by eye with the Scale magnifying glass 10X.

The international standard preparations are dissolved in PBSA at the appropriate concentration, and 10% Zwittergent is added to have a final concentration of 1%. After 30 min incubation (for the Zwittergent to react with the samples) the standard curve can be obtained via serial dilution. Samples are prediluted in PBSA and Zwittergent is added at a final concentration of 1%. After 30 minute incubation a serial dilution is performed to obtain different sample concentrations to be tested.

To prepare the agarose gel used for the assay, the agar solution must be melted and then cooled down to 60°C. A proper amount of serum (depending on the strain and according to NIBSC certificate) must be added and the solution needs to be transferred in the gel chamber. After gel formation, the wells are obtained with a cutting cylinder.

### Test procedure:

Samples are pipetted in duplicate in the wells, which are then incubated for 18-24 hours in a humid chamber. The gel is then rinsed with distilled water and then soaked in the staining solution for 15 min. After that the gel is soaked in the destaining solution for 4 minutes, dried for 12 hours, and then it is ready to be evaluated. Evaluation is performed on the scanned image of the gel with Immulab software. The measures of the circle diameters are transferred to the Combistats software for quantification.

### Zeta sizer measurements

The virosome diameter average size is measured with Malvern's Z-sizer nano ZS, in order to check the quality of virosomes upon stressed conditions and storage. Undiluted samples are loaded on a Z-size cuvette and directly measured with a He-Ne Laser (λ = 633nm) and a 173° forward detector.

The instrument measure an intrinsic property of the particles based on brownian motions in a defined fluid: neither internal control nor standard curves is needed. The average diameter is measured applying cumulant analysis fit (as defined by International Standard ISO13321:1996).

Temperature profiling has been performed on the Malvern Z-sizer ZS, to ramp the temperature from 35 to 75°C, built following instrument instruction while measuring particle size with the same parameters as described above.

The NaCl titration for cell cultured A/Victoria virosomes has been performed on the Malvern Z-sizer ZS, to dilute the salt NaCl concentration from 130 to 33 mM, built following instrument instruction while measuring particle size with the same parameters as described above.

The present invention relates to liquid compositions that stabilize virosomes and to related pharmaceutical products, preferably for use in gene therapy and/or vaccine applications. A preferred stabilized virosome containing composition disclosed herein is a liquid composition, which shows improved virosomal stability when stored in about the 2-8°C range and resistance to accidental freezing or heating while also being compatible with parenteral administration. These compositions can however also be stored at lower temperatures, e.g. -20°C or lower, -40°C or lower, -65°C or lower, -80°C or lower. They may also be more stable at temperatures above 8°C, e.g. 25°C or even higher. These compositions which are able to stabilize virosomes comprise a combined KH₂PO₄/Na₂HPO₄ buffer, and trehalose, wherein the trehalose concentration is ranging between 2% w/w and 10% w/w, as a stabilizer, which enhances the thermal stability of the virosomes. The pH of said buffer lies between 6.5 and 8. According to the present invention the liquid composition has a pH ranging between 6.5 and 8; comprises a KH₂PO₄/Na₂HPO₄ buffer, wherein the phosphate concentration is ranging between 15 Mm and 30 mM; comprises a salt at a concentration higher than 60 mM and further comprises trehalose at a concentration ranging between 2% w/w and 10% w/w. Unexpectedly, said combination has proven to be an outstanding composition for the preservation of quantity and quality of virosomes. The compositions of the present invention provide stability to virosomes at varying degrees of concentration and may be administered to a variety of vertebrate organisms, preferably mammals and especially humans. The stabilized formulations of the present invention are virosome-based compositions, which can for instance be administered as a vaccine that may offer a prophylactic advantage, against e.g. Influenza, to previously uninfected individuals.

A preferred aspect of the invention is a virosome containing composition which shows enhanced stability characteristics described herein with a virosome embedded HA concentration in the range from 25 µg/mL to 300 µg/mL. A more preferred range is from 25 µg/mL to 100 µg/mL, with an especially preferred HA concentration being from 30 to 40 µg/mL. Prophylactic compositions of the formulations of the present invention can be administered to an individual in amounts sufficient to prevent the respective disorder. The effective amount for human administration may vary according to a variety of factors such as the individual's condition, weight, sex and age. Other factors include the mode of administration. The phosphate concentration in the KH₂PO₄/Na₂HPO₄ buffer is ranging between 15 mM and 30 mM, e.g. between 15 mM and 25 mM, e.g. 20 mM.

Another essential component in these formulations which contributes to virosome stabilization over large temperature ranges and for prolonged storage periods is trehalose. The composition according to the present invention is buffered with KH₂PO₄/Na₂HPO₄ to a pH ranging between 6.5 and 8 and comprises trehalose at a concentration between 2% w/w to 10% w/ w, e.g. between 3% w/w to 8% w/w, e.g. between 3% w/w to 5% w/w e.g. 4% w/w. In a preferred embodiment of the present invention the composition is buffered with a KH₂PO₄/Na₂HPO₄ buffer comprising 20 mM phosphate to a pH ranging between 7 and 7.8 and comprises trehalose at a concentration ranging between 2% w/w to 6% w/w. In a preferred embodiment of the present invention the composition is buffered with a KH₂PO₄/Na₂HPO₄ buffer comprising 20 mM phosphate to a pH of 7.5; and trehalose is present at a concentration of 4% w/w. It was shown herein that virosomes containing antigens from cell derived A/Victoria Flu strains, a minimum concentration of 60 mM NaCl is required to keep the virosome size within the acceptable range (300 mM) suggesting that a minimum NaCl concentration of 65 mM should be added to the final composition to increase virosome stability.

Therefore, the salt concentration in the compositions according to the present invention should exceed 60 mM. In a preferred embodiment the salt concentration is ranging e.g. between 60 and 85 mM, e.g. between 65 mM and 75 mM, e.g. between 65 and 70 mM, e.g. 65 mM.

This amount of salt will be sufficient to reach the isotonicity of the formulation. In a preferred embodiment said salt is NaCl. Other types of salt known in the art are equally suited for the formulations according to the present invention. The skilled person would know which salts to select.

In view of the discussion above, the present invention relates to liquid compositions containing a virosome, that can e.g. be used in gene therapy and/or gene vaccination applications, which show improved stability properties and which at least contain a KH₂PO₄/Na₂HPO₄ buffer at a pH between 6.5 and 8, wherein the phosphate concentration is ranging between 15 and 30 mM; trehalose, wherein the trehalose concentration is ranging between 2% w/w and 10% w/w ; and further comprises a salt at a concentration between 60 and 85 mM.

In a preferred embodiment of the present invention the composition is buffered with a KH₂PO₄/Na₂HPO₄ buffer to a pH of 7.5, wherein the phosphate concentration is 20 mM; and trehalose is present at a concentration of 4% w/w and further comprises NaCl at a concentration between 60 and 85 mM. In a preferred embodiment of the just described composition NaCl is present at a concentration of 65 mM. In one embodiment the compositions according to the present invention are contained in a vial such as e.g. DIN 2R type I borosilicate glass vial. In another embodiment, the formulations are contained in a bag. Bags that contain the formulations of the present invention may comprise layers made of e.g. Ethylene Vinyl Acetate Copolymer (EVA) or Ethyl Vinyl Alcohol (EVOH). In yet another embodiment of the present invention, the formulations are contained in a syringe such as e.g. a glass, polypropylene or polycarbonate pre-filled syringe.

The virosome formulations described herein can be administered to the vertebrate host (preferably a mammalian host and especially a human recipient) by any means known in the art, such as parenteral or nasal routes.

In accordance with the formulations disclosed herein, also disclosed are methods of preserving a virosome which comprise preparing virosome containing compositions according to any one of claims 1-5, such formulations which result in improved virosomal stability when stored below -65 °C and in about the 2-8 °C range and possibly higher while also being compatible with parenteral administration, especially parenteral administration to humans.

Another aspect of the present invention therefore relates to methods of preserving a virosome which comprise preparing a composition according to any one of claims 1-5 and storing said composition at a temperature ranging between 2°C and 8°C.

The following examples are provided to illustrate the present invention without, however, limiting the same hereto.

### EXAMPLES

### Example 1

### Experimental design and methodology.

Three different Influeza derived virosome preparations each comprising HA molecules from a different Influenza strain have been prepared in a control composition and rebuffered in 8 experimental formulations (**Table 1**) with a "Cogent µSCALE TFF system" UF/DF instrument (Millipore). Eluates were filter sterilized (0.22 µm) and aliquoted in glass vials (3 mL per vial). The control composition is the currently marketed Inflexal®V composition which is buffered with 50 mM KH₂PO₄/Na₂HPO₄ to a pH of 7.4; and which comprises NaCl at a concentration of about 82 mM.

Subsequently, vials were subjected to 1 cycle of freeze/thawing (F/T) or were incubated at 5±3°C for a stability study (during one and three months), both simulating real-time conditions. Vials were stored together with their respective controls in aliquots at 5±3°C until sample analysis was performed in duplicate. Samples were analyzed by: single radial immunodiffusion (SRID) for HA quantification and Z-sizer nano (ZS) for virosome particle diameter measurement as described above in the description.

**Table 1. List of formulations selected for this study.**

| Buffering species | buffer strength | pH | Stabilizer | Stabilizer concentration |
|---|---|---|---|---|
| KH₂PO₄/ Na₂HPO₄ | 20 mM | 7.0 | mannitol | 4% |
| KH₂PO₄/ Na₂HPO₄ | 20 mM | 7.5 | trehalose | 8% |
| KH₂PO₄/ Na₂HPO₄ | 20 mM | 7.0 | none | N.A. |
| KH₂PO₄/ Na₂HPO₄ | 20 mM | 7.0 | trehalose | 8% |
| KH₂PO₄/ Na₂HPO₄ | 20 mM | 7.5 | none | N.A. |
| KH₂PO₄/ Na₂HPO₄ | 20 mM | 7.5 | sucrose | 8% |
| KH₂PO₄/ Na₂HPO₄ | 20 mM | 7.0 | sucrose | 8% |
| KH₂PO₄/ Na₂HPO₄ | 20 mM | 7.5 | mannitol | 4% |
| | | | | |
| Control Formulation | 53 mM | 7.4 | NaCl | 82 mM |

### Results and conclusion.

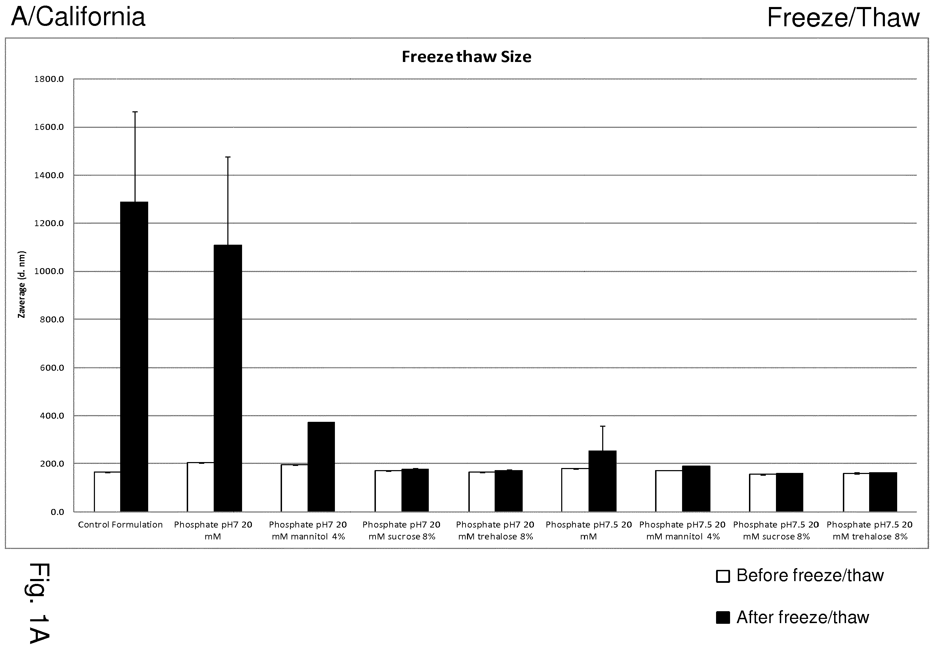

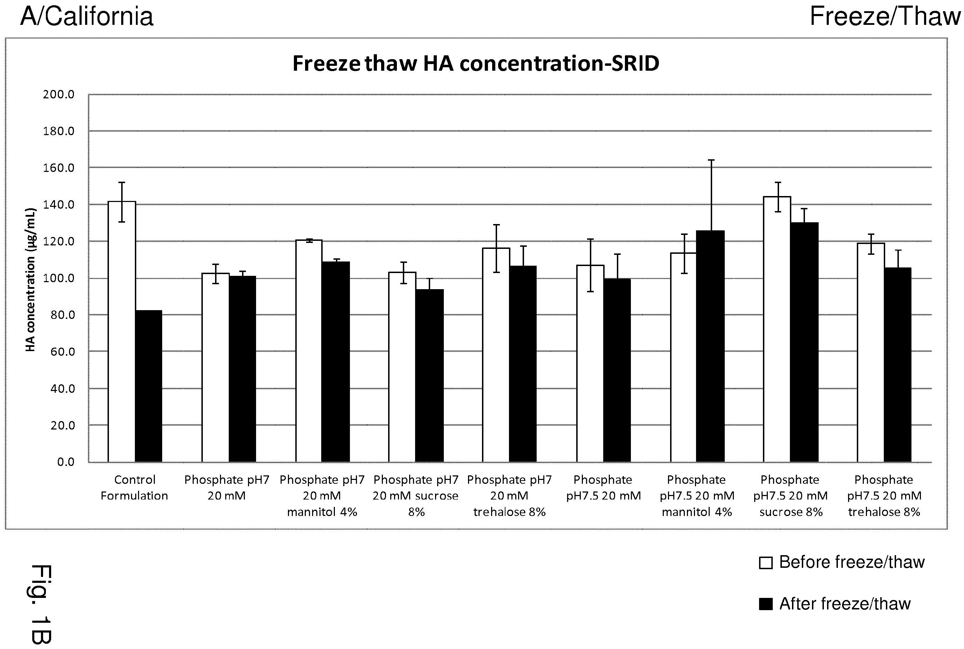

Average diameter size and HA concentration have been measured for A/California derived virosomes before and after one freeze/thaw cycle (Fig.1). It was observed that after one freeze/thaw cycle, the control composition is detrimental for virosome stability both in terms of diameter size (Fig. 1A) and HA concentration (Fig. 1B).
Unexpectedly, excellent performance was observed in terms of average diameter size for the experimental formulations tested and a clear added value was observed when adding a mono-or disaccharide, especially at pH 7.0 (Fig. 1A). Indeed, in these formulations, no significant change in virosome size was measured by Dynamic Light Scattering. A good stability profile was observed for the experimental formulations tested in terms of HA concentration variation after freeze/thawing (Fig. 1B).

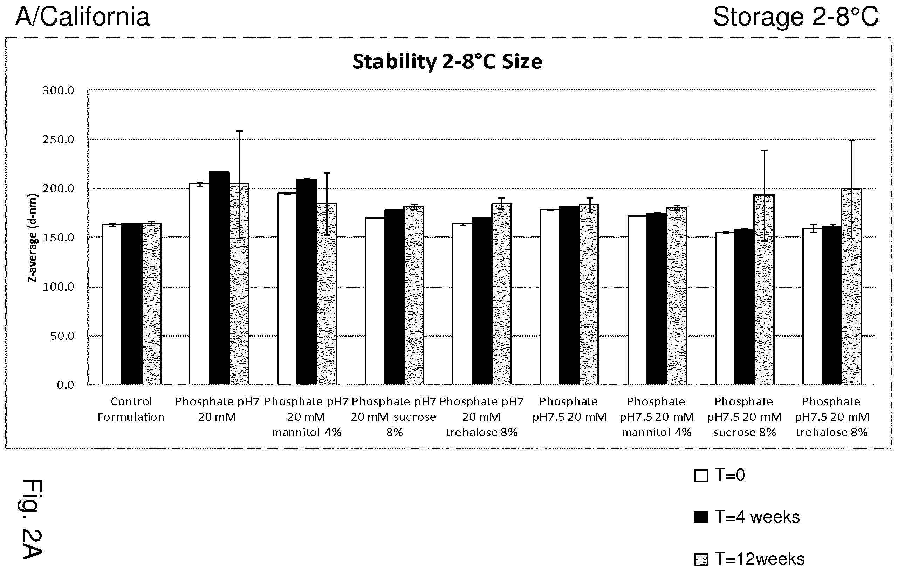

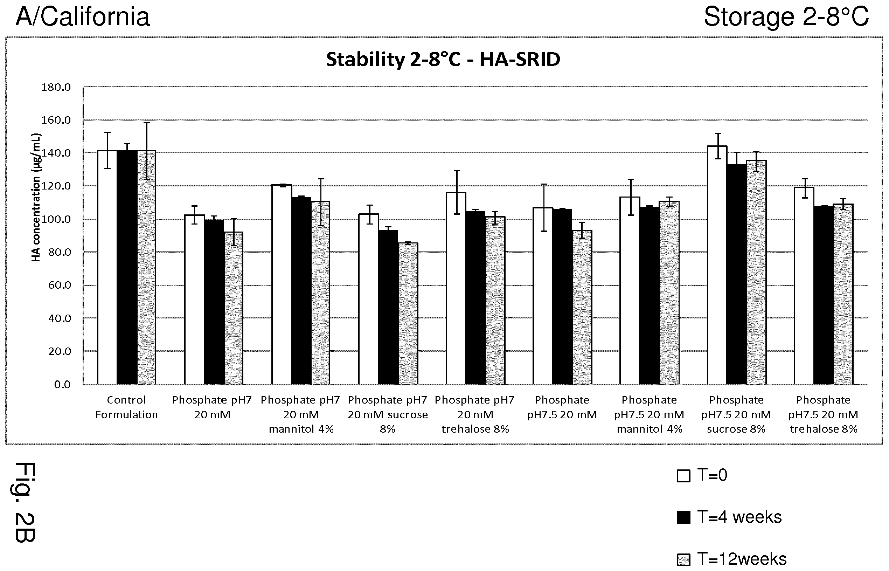

A/California derived virosomes were stored at 5±3°C for 12 weeks and the average diameter size and HA concentration have been measured at t=0, t= 4 ant t=12 weeks (Fig. 2). No significant variation was observed over time in terms of virosome size for all the formulations analyzed (Fig. 2A). The same trend of HA concentration variation over time was observed for the control composition and the experimental formulations tested (Fig. 2B).

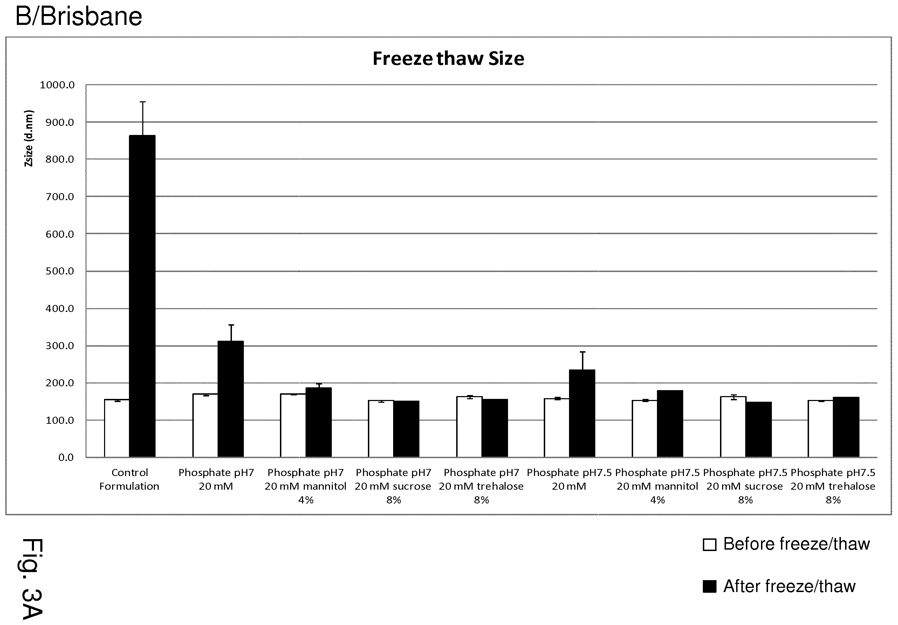

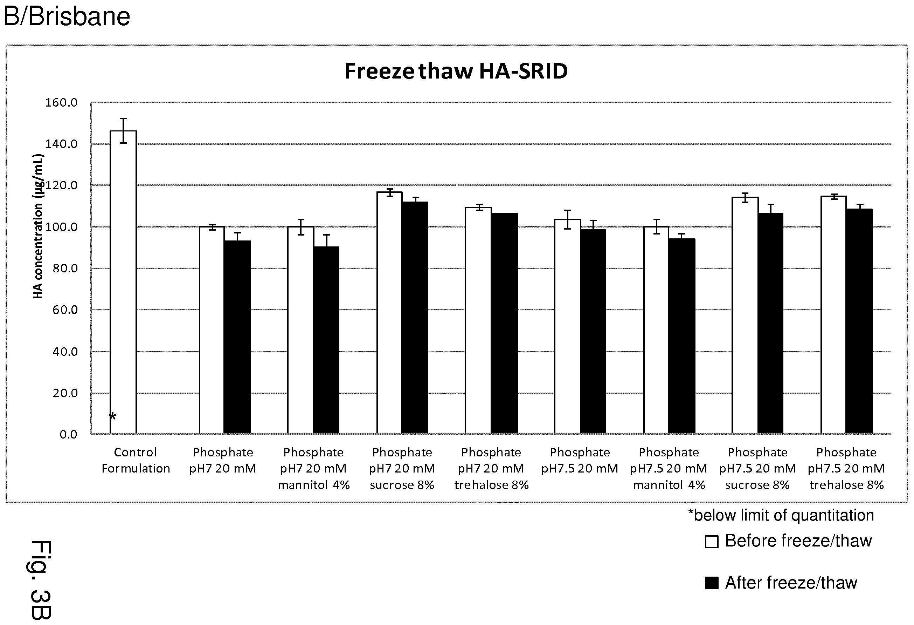

Average diameter size and HA concentration have been measured for B/Brisbane derived virosomes before and after one freeze/thaw cycle (Fig.3). It was observed that after one freeze/thaw cycle, the control composition is detrimental for virosome stability both in terms of diameter size (Fig. 3A) and HA concentration (Fig. 3B).

Unexpectedly, excellent performance was observed both in terms of average size (Fig.3A) and HA concentration (Fig.3B) for the alternative formulations tested: no significant variation in virosome diameter size nor HA concentration was measured after freeze/thawing. A clear added value in preventing diameter size variations after freeze/thawing was observed when adding a mono- or disaccharide.

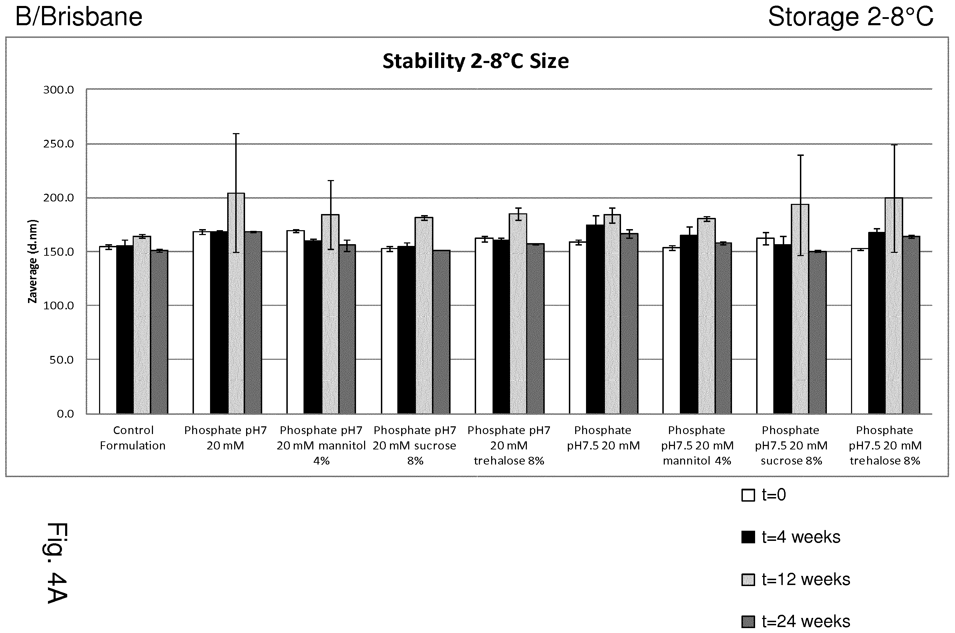

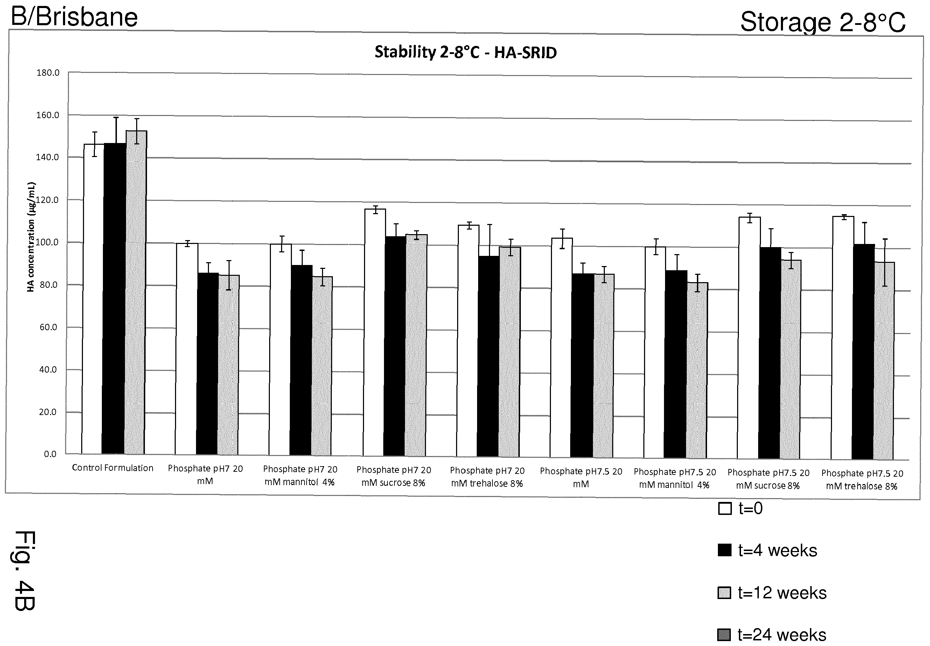

B/Brisbane derived virosomes were stored at 5±3°C for 24 weeks and average diameter size was measured at t=0, t= 4, t=12 and t=24 weeks and HA concentration was measured at t=0 t= 4 and t= 12 (Fig.4). No significant variation was observed over time in terms of virosome diameter size (Fig.4A) and in terms of HA concentration (Fig.4B) for all the formulations analyzed. Taken together, this data suggests a good comparability of the experimental formulations with the control formulation.

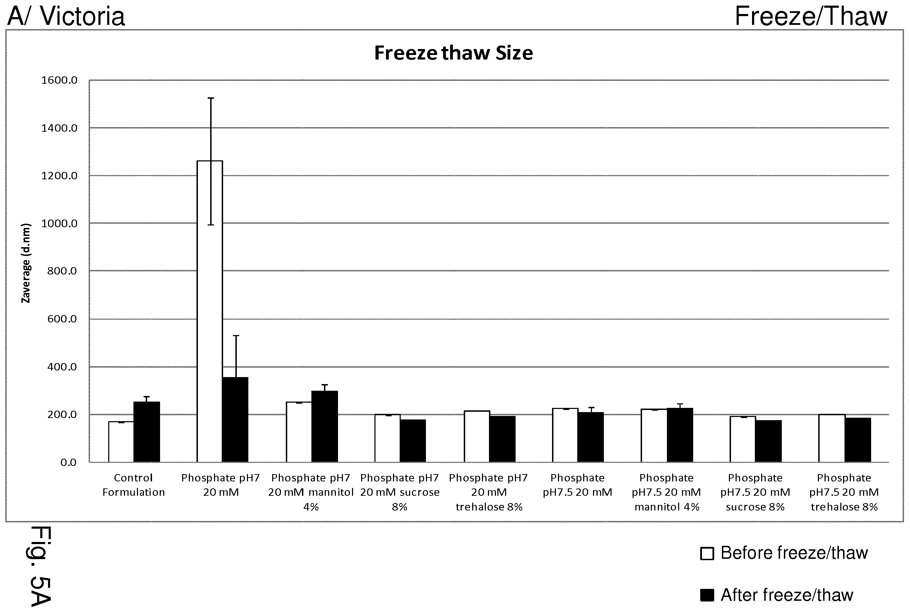

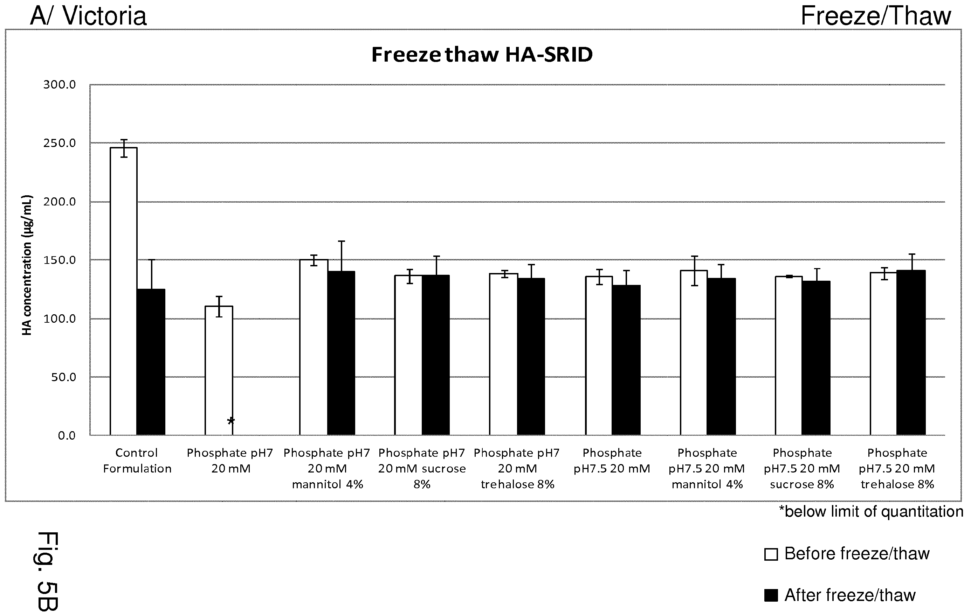

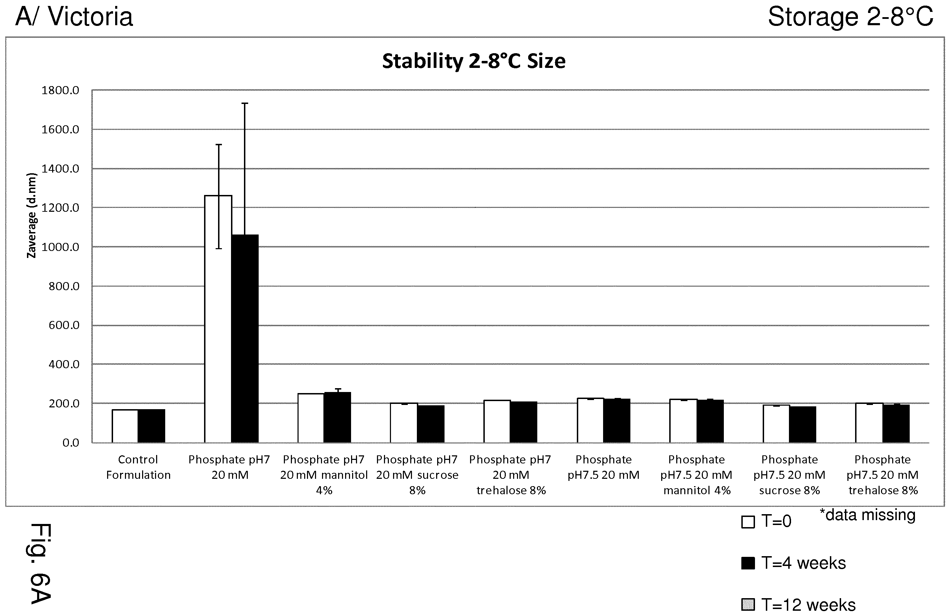

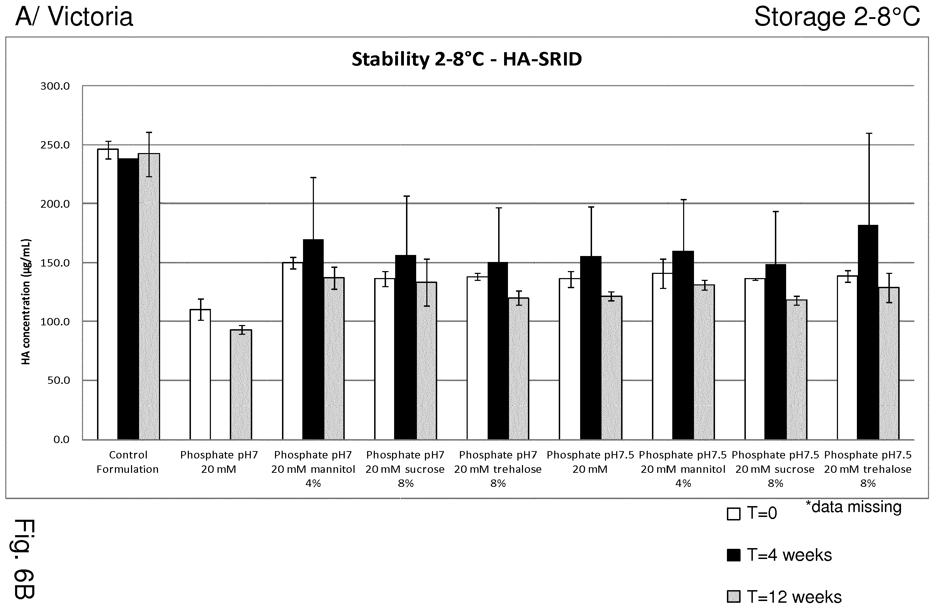

Average diameter size and HA concentration have been measured for A/Victoria derived virosomes before and after one freeze/thaw cycle (Fig.5). The control composition was clearly suboptimal in ensuring stability after this stressor, in particular in terms of HA concentration. Good performance was observed in terms of average size for all the experimental formulations tested (except for the sugar free phosphate pH 7.0) (Fig.5A): no significant variation in virosome diameter size was measured via Dynamic Light Scattering. Unexpectedly, excellent performance was observed for all the experimental formulations comprising a mono- or disaccharide, in terms of HA concentration variation after freeze/thawing (Fig.5B). A/Victoria derived virosomes were stored at 5±3°C for 12 weeks and average diameter size was measured at t=0 and t= 4 weeks and HA concentration was measured at t=0, t= 4 ant t=12 weeks (Fig.6). No significant variation was observed over time in terms of virosome size (Fig.6A) and in terms of HA concentration (Fig.6B) for all the experimental formulations analyzed except for the sugar free phosphate pH 7.0, showing the added value of mono- or disaccharide to this buffer. Taken together, this data suggests a good comparability of the alternative formulations with the control formulation.

The freeze/thaw experiments (Figs. 1, 3 and 5) show that the formulations according to the present invention are surprisingly very well suited to protect virosomes from an incidental freezing event and thereby provide more stability to the virosomes.

Taken everything together, the stability studies (12 weeks at 5±3°C) wherein the average diameter size and HA concentration have been measured (Figs. 2, 4 and 6), show good comparability between the experimental formulations and the control formulation, which is a composition that has proven to be robust and efficient for many years already and is currently still used on the market for preserving virosomes.

Combining all data obtained as described above and after statistical analysis, a specific combination of variables has clearly demonstrated to provide the best stability for virosomes. The composition that proved to be most robust for virosomal stability was a buffered composition comprising 20 mM Na₂HPO₄/KH₂PO₄ at a pH of 7.5 and further comprising 8% trehalose.

### Example 2

### Experimental design and methodology.

A temperature profile has been obtained by measuring virosome diameter size variation over temperature to identify aggregation onset and overall size variation upon temperature increase, in the different formulations tested. A temperature ramp from 35°C to 75°C was obtained with the Z-sizer ZS (Malvern) and virosome size was measured every 3°C directly in the same cuvette.

### Results and conclusion.

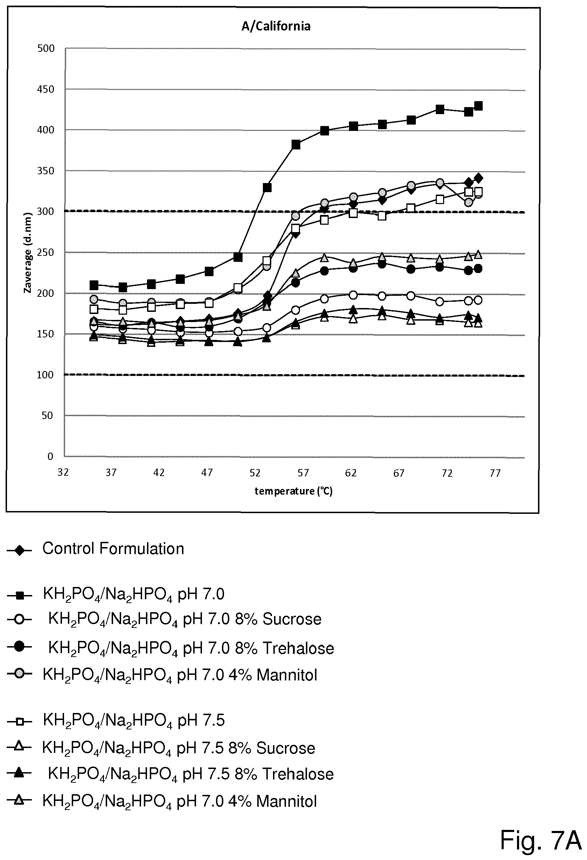

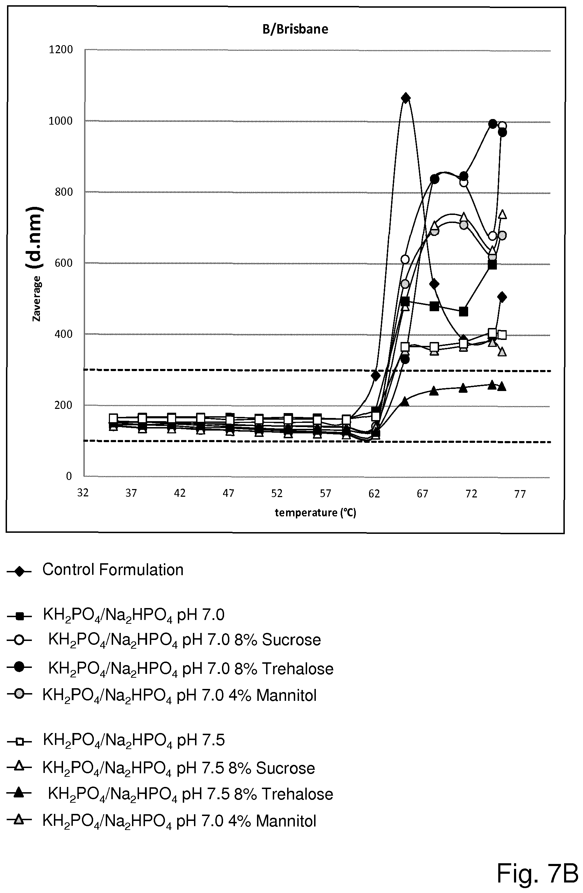

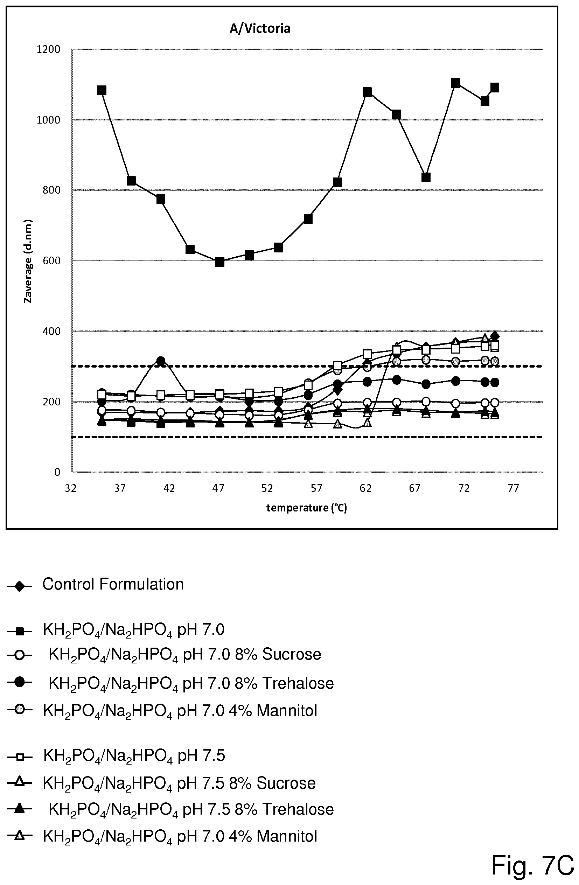

The temperature dependent profile, which indicates the aggregation onset temperature, and the overall size variation of virosomes in the different formulations tested are shown in Figs.7(A, B and C). For all three strains analyzed, the control composition was suboptimal for ensuring thermal stability after accelerated temperature stress. Indeed, for all strains, the virosome diameter size increased dramatically (up to above 300 nm) in the temperature range between 52°C and 62°C. For B/Brisbane (Fig.7B) almost all the new formulations tested increased the onset temperature, and the trehalose containing composition at pH 7.5 had a positive effect on the maximum size reached at high temperatures, i.e. the maximum diameter size did not supersede 300 nm.

For A/California (Fig.7A) and A/Victoria (Fig.7C), the increase in onset temperature was less pronounced, however all the new formulations tested surprisingly showed a clear positive effect in reducing the increase in diameter size of the virosomes.

The final outcome of this experiment confirmed the results obtained in Example 1, suggesting that a composition buffered with 20 mM Na₂HPO₄/KH₂PO₄ at a pH of 7.5 and further comprising 8% trehalose is the most promising combination for increasing virosome stability.

### Example 3

### Experimental design and methodology.

A titration profile was performed on cell-cultured A/Victoria derived virosomes measuring virosome size variation over salt concentration to identify aggregation onset and overall size variation upon salt concentration decrease. A salt (NaCl) concentration ramp from 130 to 33 mM was performed in the Z-sizer ZS (Malvern) and virosome size was measured nineteen times in the same cuvette at decreasing NaCl concentrations.

### Results and conclusion.

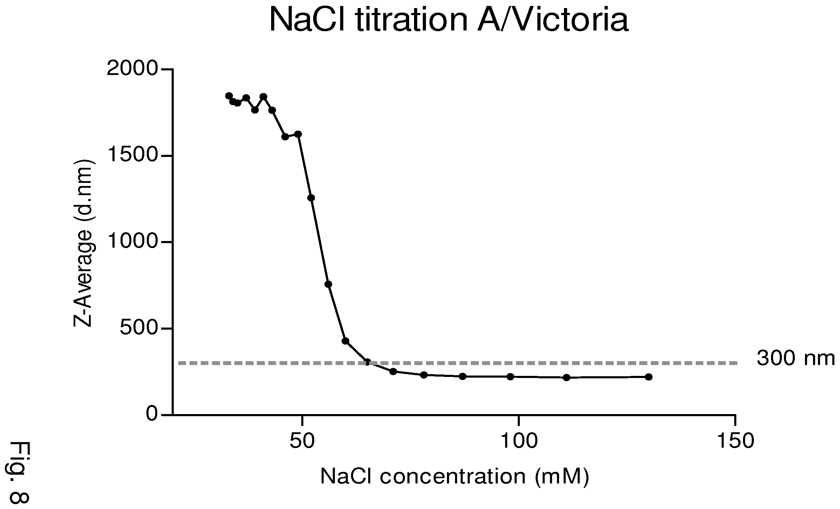

The NaCl concentration dependent profile, which indicates the virosome aggregation onset at a certain NaCl concentration, and the overall size variation of A/Victoria virosomes is shown in Fig.8.

The NaCl concentration was decreased from 130 to 33 mM, by automatically diluting a composition containing 20 mM Na₂HPO₄/KH₂PO₄ at a pH of 7.5 and 130 mM NaCl. A/Victoria virosome diameter size increased dramatically (up to above 300 nm) in the NaCl concentration below 65 mM. The same effect was observed in a similar experiment where 8% trehalose was added (data not shown).

The final outcome of this experiment showed that for cell derived A/Victoria strain a minimum concentration of 65 mM NaCl is required to keep the virosome size within the acceptable range (300 mM) suggesting that a minimum NaCl concentration of 65 mM should be added to the final composition to increase virosome stability.

### Example 4

### Experimental design and methodology.

Three different Influenza derived virosome preparations each comprising HA molecules from a different Influenza strain have been prepared in a control formulation and rebuffered in 2 experimental formulations (Table 2) with a "Cogent µSCALE TFF system" UF/DF instrument (Millipore). The control composition was also rebuffered with the same system, to exclude buffer exchange effects.

**Table 2. Composition of the experimental buffers used.**

| Buffer Name | KH₂PO₄/Na₂HPO₄ (mM) | pH | Trehalose (% w/w) | Sucrose (% w/w) | NaCl (mM) |
|---|---|---|---|---|---|
| Buffer T | 20 | 7.5 | 4 | | 75 |
| Buffer S | 20 | 7.5 | | 4 | 75 |

Eluates were filter sterilized (0.22 µm) and aliquoted in glass vials (3 mL per vial) or pooled into a trivalent product and then aliquoted in glass vials. Said trivalent product (also abbreviated to "trivalent") is a mixture of three different virosomes each containing antigens from a different influenza strain. In the present experiment, a trivalent contains three different virosomes having influenze antigens from either: the A/Victoria strain, the B/Brisbane strain or the A/California.

The control composition is the one of the currently marketed Inflexal®V composition which is buffered with 50 mM KH₂PO₄/Na₂HPO₄ to a pH of 7.4; and which comprises NaCl at a concentration of about 82 mM. Subsequently, vials were subjected to 1 cycle of freeze/thawing (F/T) or were incubated at 5±3°C for a stability study (during one and three months), both simulating real time conditions. Vials were stored together with their respective controls in aliquots at 5±3°C until sample analysis was performed in triplicate. Samples were analyzed by: single radial immunodiffusion (SRID) for HA quantification and Z-sizer nano (ZS) for virosome particle diameter measurement as described above.

### Results and conclusion.

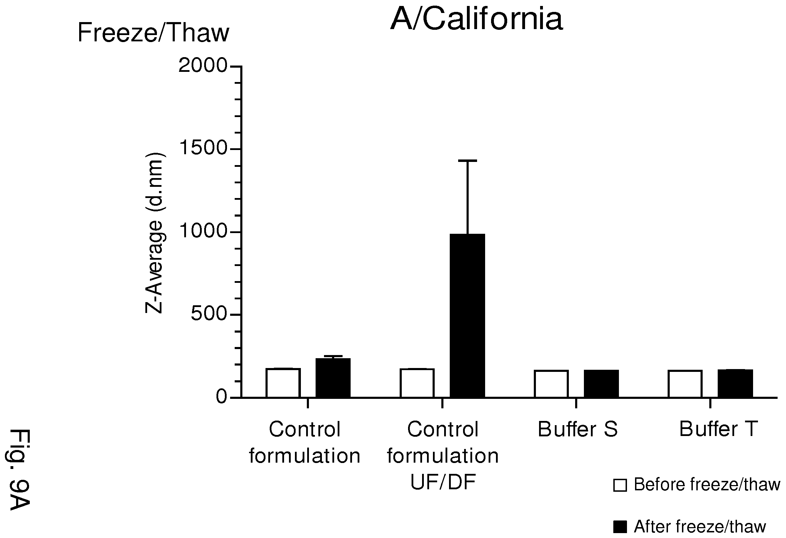

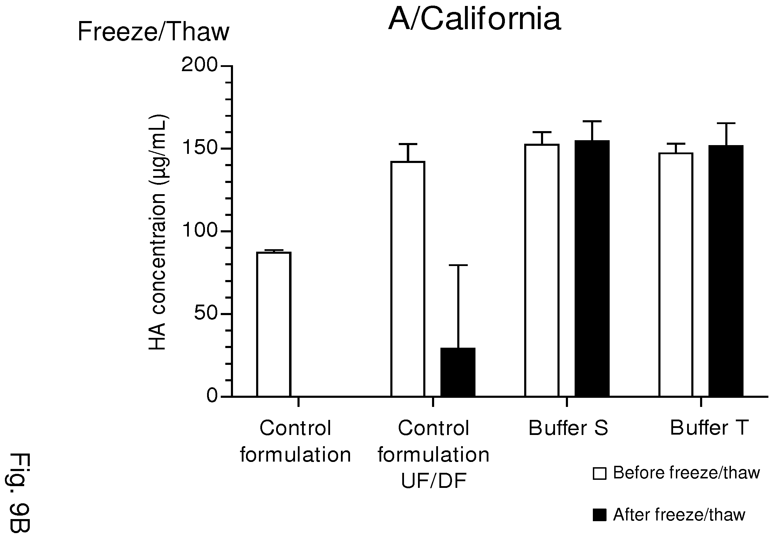

Average diameter size and HA concentration have been measured for cell derived A/California virosomes before and after one freeze/thaw cycle (Fig. 9). It was observed that after one freeze/thaw cycle, the control formulations are detrimental for virosome stability both in terms of diameter size (Fig. 9A) and HA concentration (Fig. 9B).

Unexpectedly, excellent performance was observed in terms of average diameter size for the experimental formulations tested (Buffer T and Buffer S) and a clear added value was observed when adding a disaccharide (Fig. 9A). Indeed, in these formulations, no significant change in virosome size was measured by Dynamic Light Scattering. A very good stability profile was observed for the experimental formulations tested in terms of HA concentration variation after freeze/thawing (Fig. 9B).

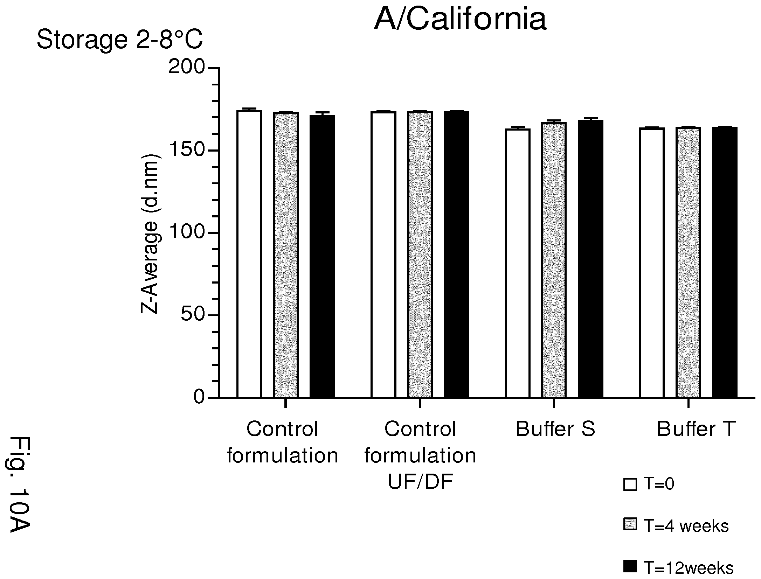

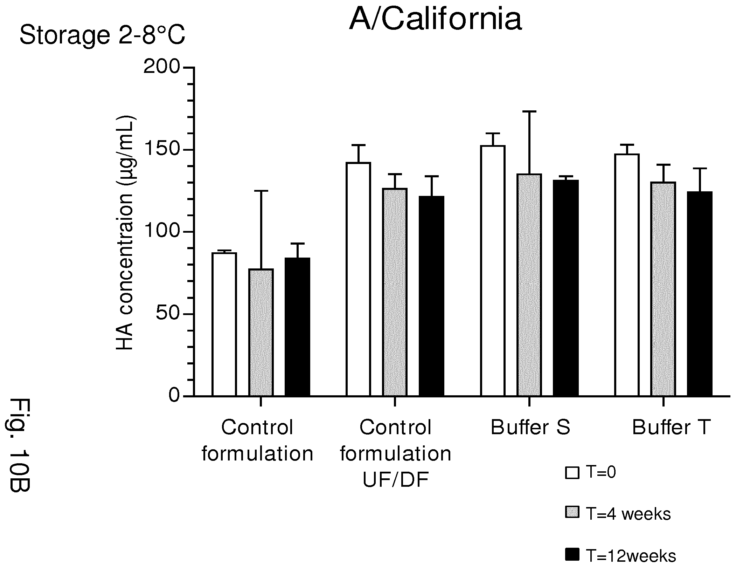

A/California cell derived virosomes were stored at 5±3°C for 12 weeks and the average diameter size and HA concentration have been measured at t=0, t= 4 ant t=12 weeks (Fig. 10). No significant variation was observed over time in terms of virosome size for all the formulations analyzed (Fig. 10A). The same trend of HA concentration variation over time was observed for the control composition and the experimental formulations tested (Fig. 10B).

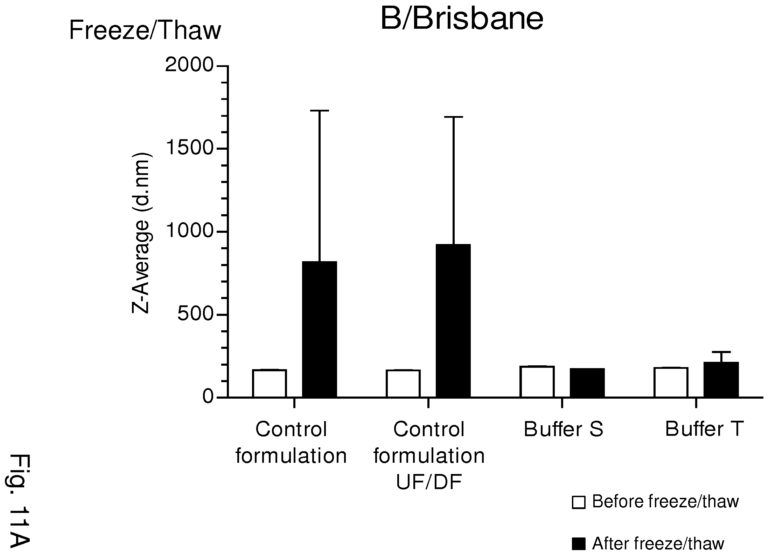

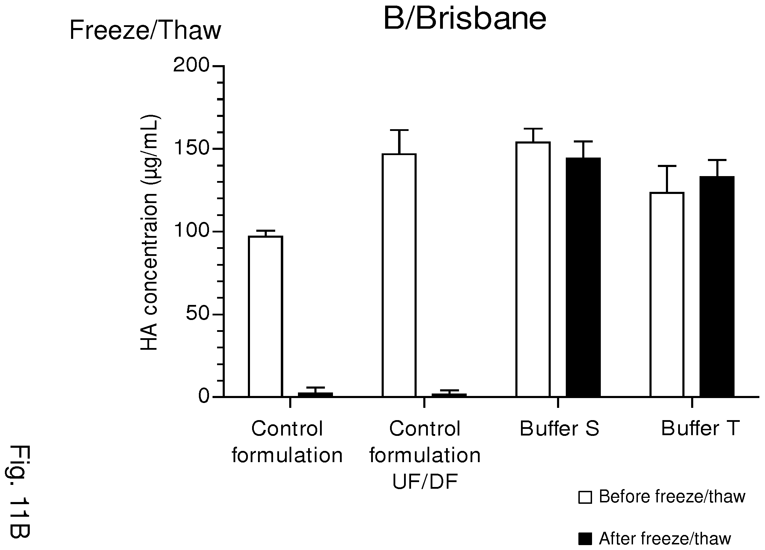

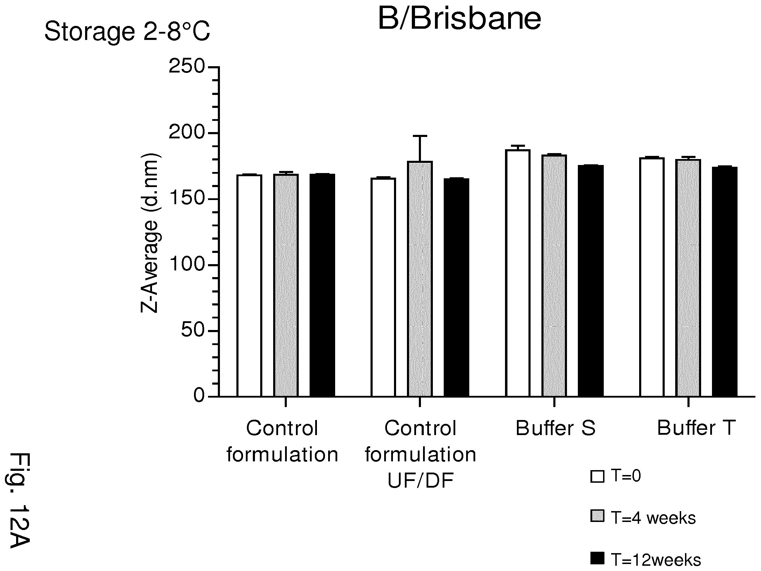

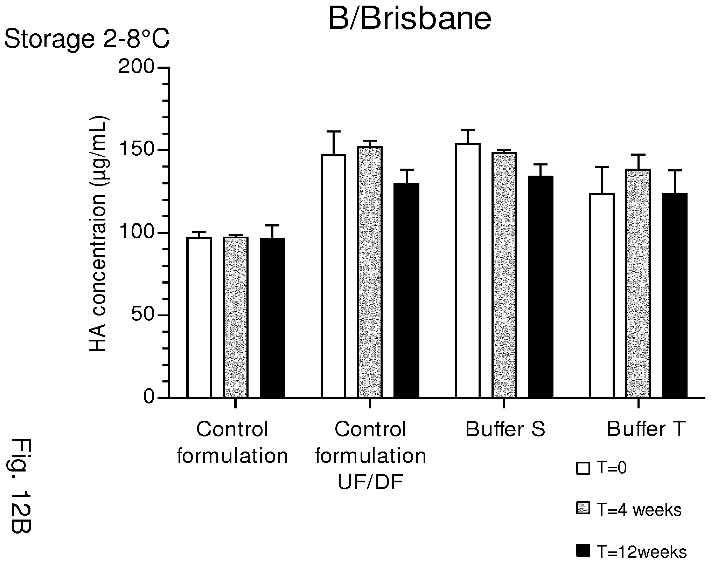

Average diameter size and HA concentration have been measured for B/Brisbane derived virosomes before and after one freeze/thaw cycle (Fig.11). It was observed that after one freeze/thaw cycle, the control formulations are detrimental for virosome stability both in terms of diameter size (Fig. 11A) and HA concentration (Fig. 11B).
Unexpectedly, excellent performance was observed both in terms of average size (Fig.11A) and HA concentration (Fig.11B) for the alternative formulations tested (Buffer T and Buffer S): no significant variation in virosome diameter size nor HA concentration was measured after freeze/thawing. A clear added value in preventing diameter size variations after freeze/thawing was observed when adding a disaccharide.
B/Brisbane derived virosomes were stored at 5±3°C for 12 weeks and average diameter size was measured at t=0, t= 4 and t=12 weeks and HA concentration was measured at t=0 t=4 and t=12 (Fig.12). No significant variation was observed over time in terms of virosome diameter size (Fig.12A) and in terms of HA concentration (Fig.12B) for all the formulations analyzed. Taken together, this data suggests a good comparability of the experimental formulations with the control composition (Fig. 12).

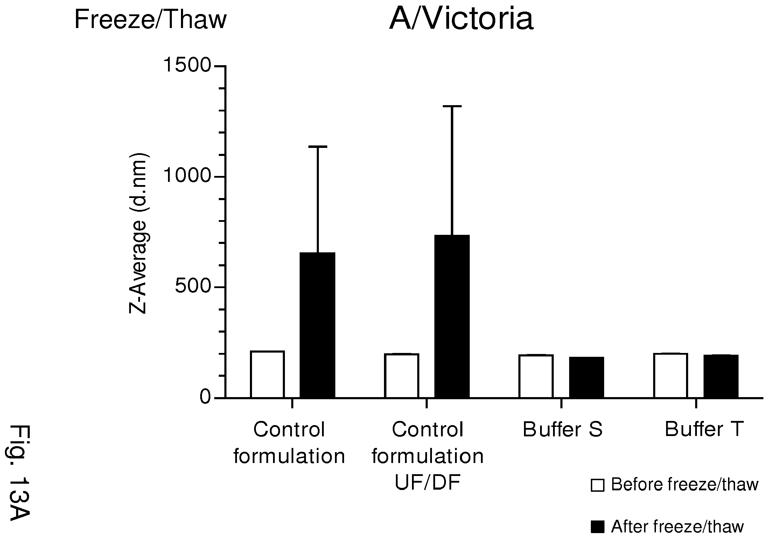

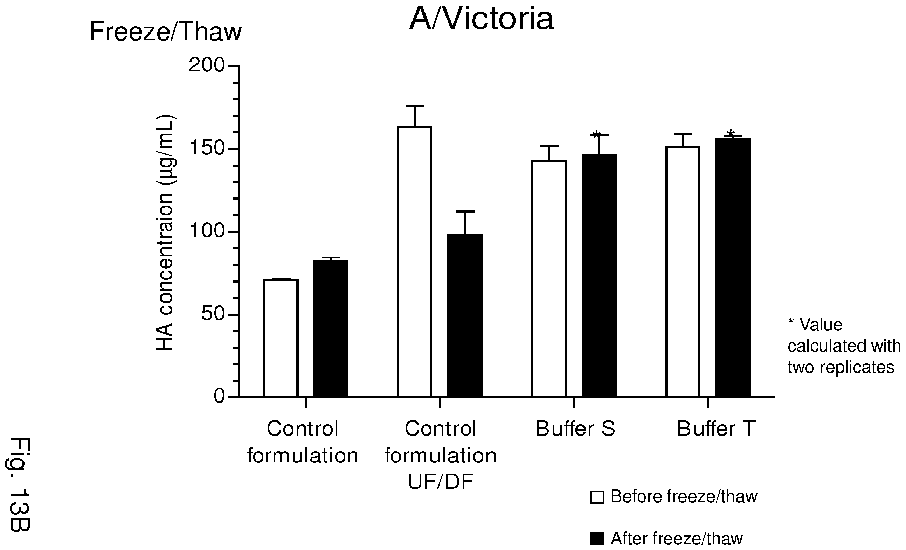

Average diameter size and HA concentration have been measured for A/Victoria derived virosomes before and after one freeze/thaw cycle (Fig. 13). The control composition was clearly suboptimal in ensuring stability after this stressor, in particular in terms of size variation. Good performance was observed in terms of average size for boht experimental formulations tested (Fig.13A): no significant variation in virosome diameter size was measured via Dynamic Light Scattering. Unexpectedly, excellent performance was observed for the experimental formulations comprising a disaccharide (Buffer T and S), in terms of HA concentration variation after freeze/thawing (Fig.13B).

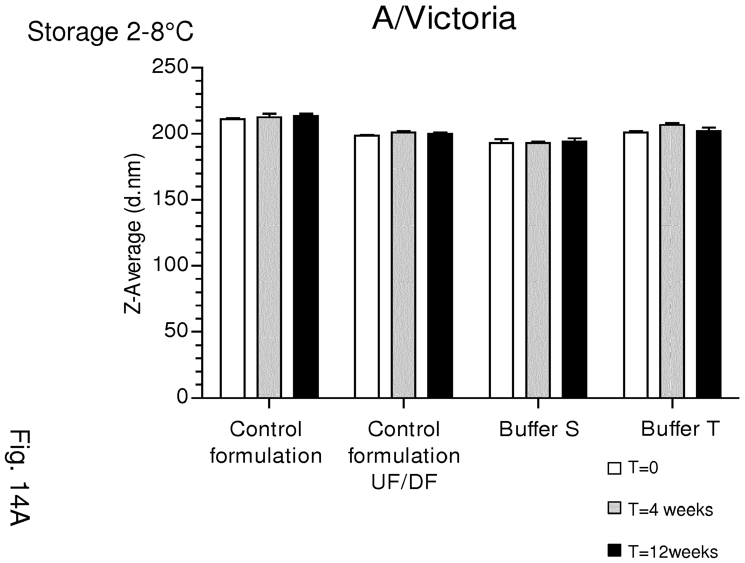

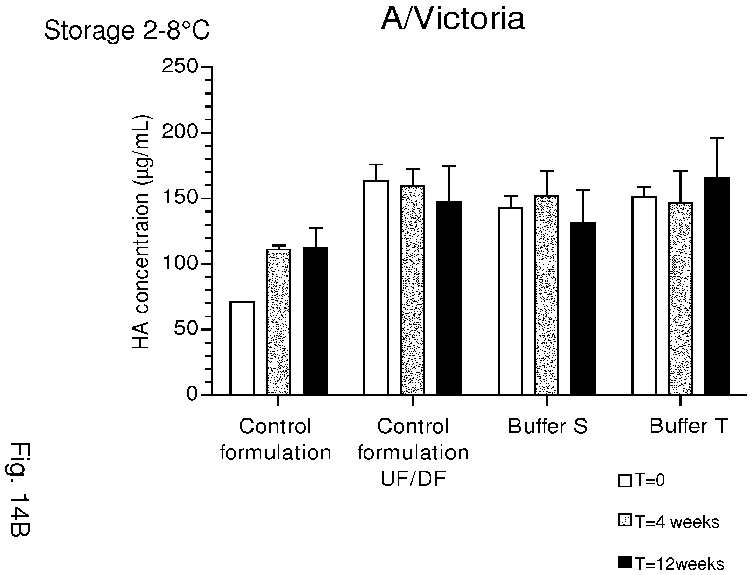

A/Victoria derived virosomes were stored at 5±3°C for 12 weeks and average diameter size and HA concentration were measured at t=0, t= 4 and T=12 weeks (Fig. 14). No significant variation was observed over time in terms of virosome size (Fig. 14A) and in terms of HA concentration (Fig. 14B) for the experimental formulations analyzed, showing the added value of disaccharide to this buffer.

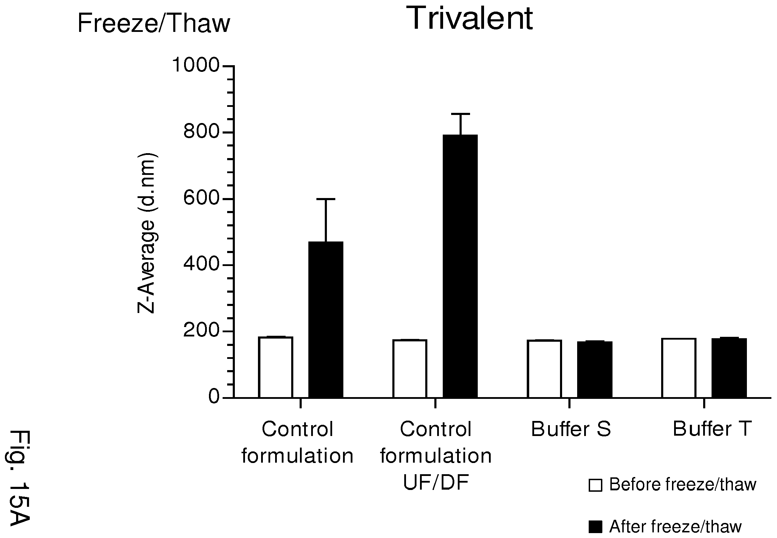

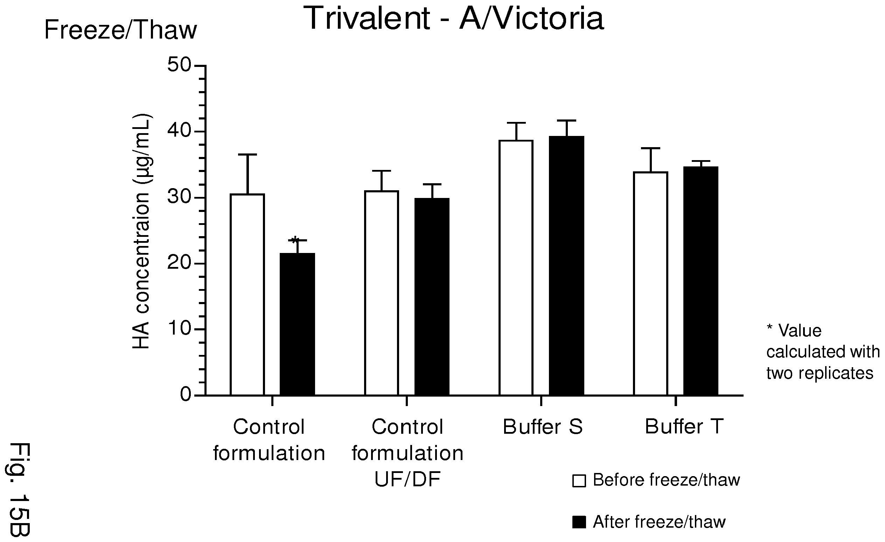

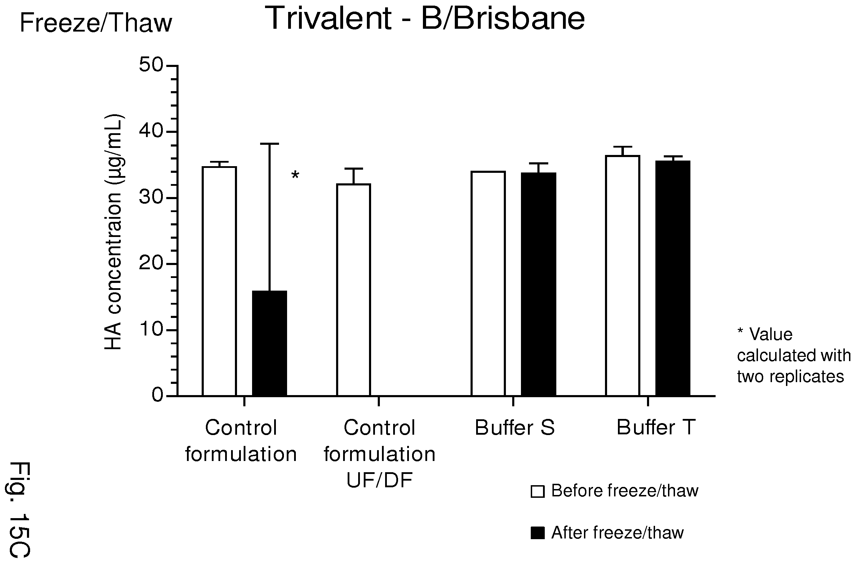

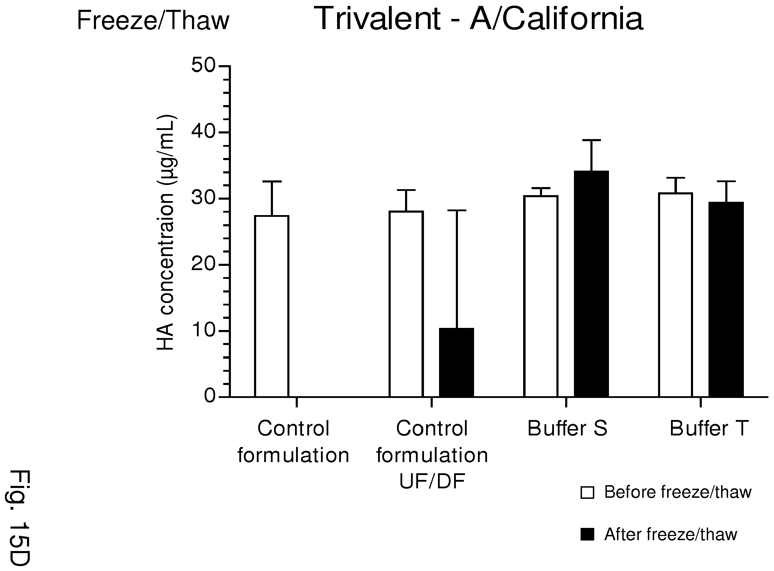

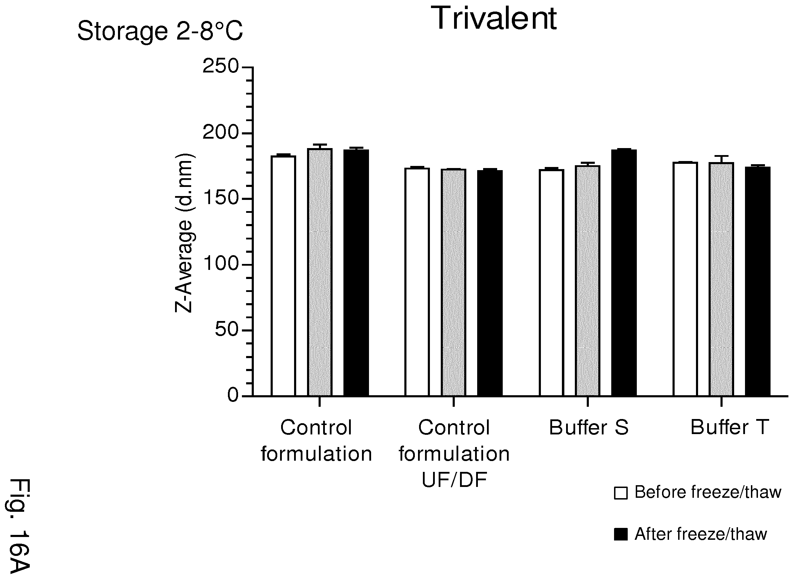

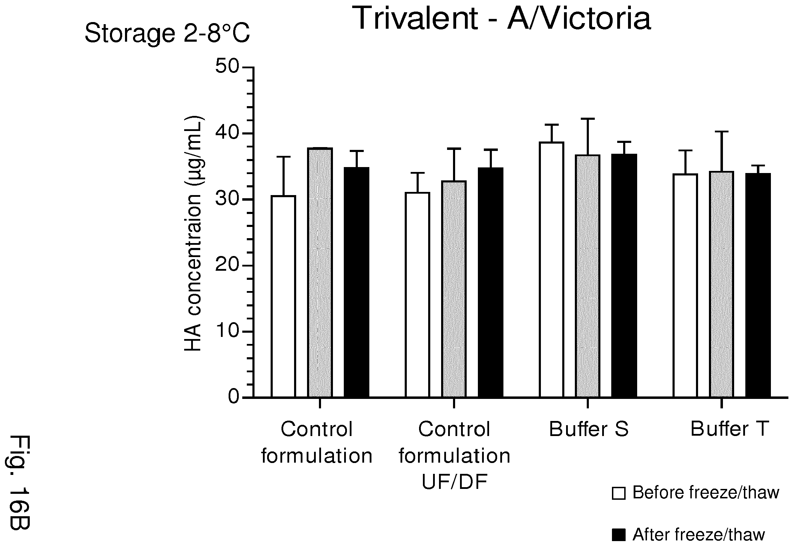

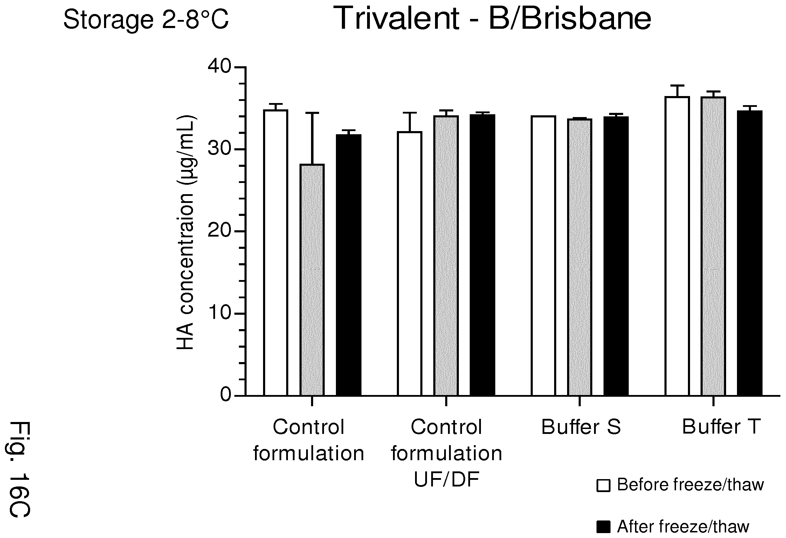

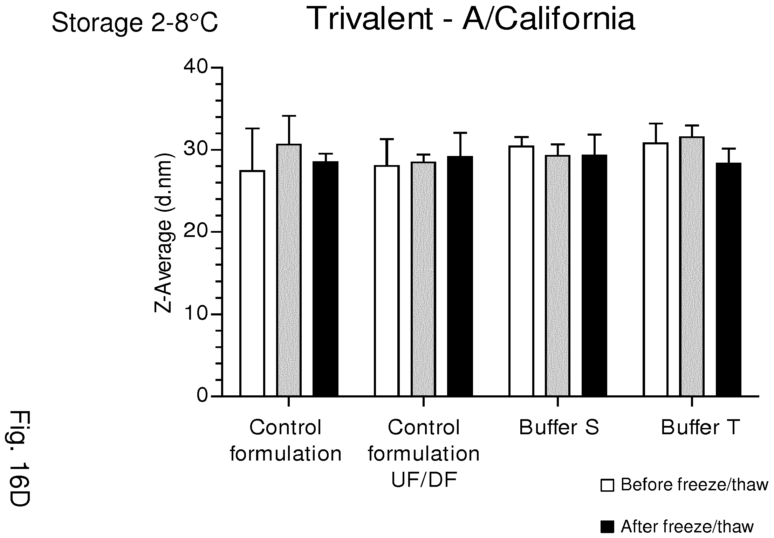

Average diameter size and HA concentration have been measured for trivalent mixtures of virosomes before and after one freeze/thaw cycle (Fig. 15). The control composition was clearly suboptimal in ensuring stability after this stressor, both in terms of HA concentration and size. Good performance was observed in terms of average size for both experimental formulations tested (Fig.15A): no significant variation in virosome diameter size was measured via Dynamic Light Scattering. Unexpectedly, excellent performance was observed for the experimental formulations comprising a disaccharide, in terms of HA concentration variation after freeze/thawing (Fig. 15B, 15C, 15D).
Trivalent virosomes were stored at 5±3°C for 12 weeks and average diameter size and HA concentration were measured at t=0, t= 4 and T=12 weeks (Fig. 16). No significant variation was observed over time in terms of virosome size (Fig. 16A) and in terms of HA concentration (Fig.16B, 16C and 16D) for the experimental formulations analyzed, showing the added value of disaccharide to this buffer.

Taken together, this data suggests a good comparability of the alternative formulations with the control formulation. The freeze/thaw experiments (Figs. 9, 11, 13 and 15) show that the formulations according to the present invention are surprisingly very well suited to protect virosomes from an accidental freezing event and thereby provide more stability to the virosomes.

Taken everything together, the stability studies (12 weeks at 5±3C) wherein the average diameter size and HA concentration have been measured (Figs. 10, 12, 14 and 16), show good comparability between the experimental formulations and the control formulation, which is a composition that has proven to be robust and efficient for many years already and is currently still used on the market for preserving virosomes.

Combining all data obtained as described above and after statistical analysis, a specific combination of variables has clearly demonstrated to provide the best stability for virosomes. The composition that proved to be most robust for virosomal stability was a buffered composition comprising 20 mM Na₂HPO₄/KH₂PO₄ at a pH of 7.5, 75 mM NaCl and further comprising 4% trehalose.

### Example 5

### Experimental design and methodology.

Three different Influenza derived virosome preparations each comprising HA molecules from a different Influenza strain have been prepared in a control formulation and rebuffered in 2 experimental formulations (as disclosed in Table 1) with a "Cogent µSCALE TFF system" UF/DF instrument (Millipore). The control composition was also rebuffered with the same system, to exclude buffer exchange effects.

Eluates were filter sterilized (0.22 µm) and pooled into a trivalent product and then aliquoted in glass vials (3 mL per vial). The control composition is the one of the currently marketed Inflexal®V composition which is buffered with 50 mM KH₂PO₄/Na₂HPO₄ to a pH of 7.4; and which comprises NaCl at a concentration of about 82 mM. Subsequently, vials were incubated at <-65°C for a stability study (during 12 weeks). Vials were stored together with their respective controls in aliquots at <-65°C until sample analysis was performed in triplicate. Samples were analyzed by: single radial immunodiffusion (SRID) for HA quantification and Z-sizer nano (ZS) for virosome particle diameter measurement as described above in the assay description.

### Results and conclusion.

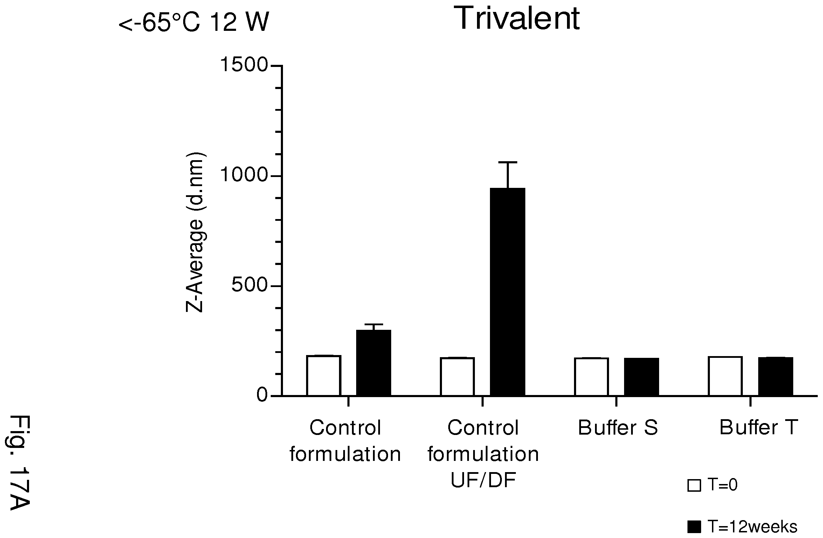

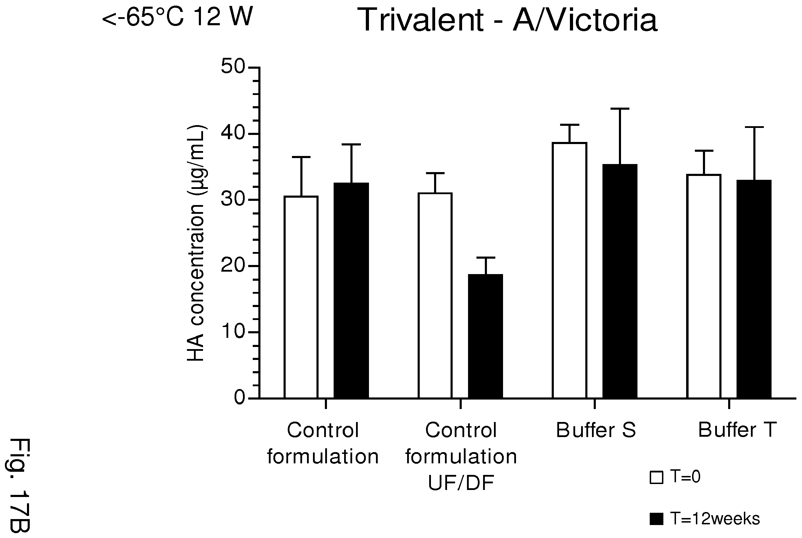

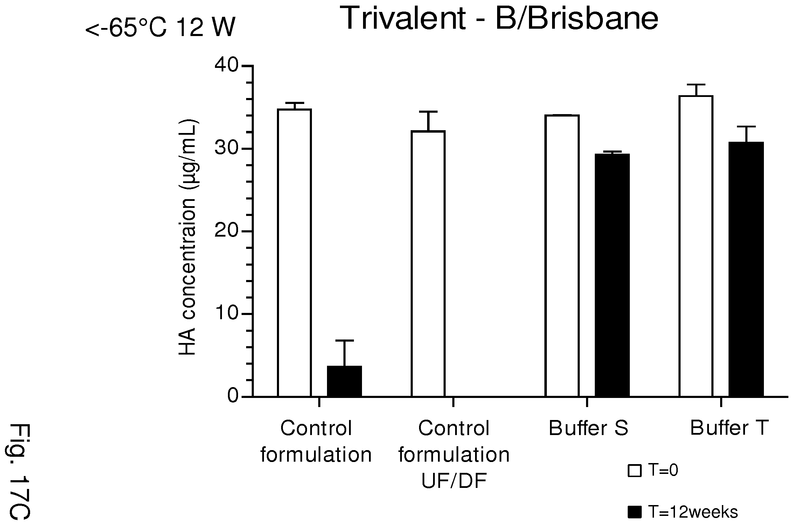

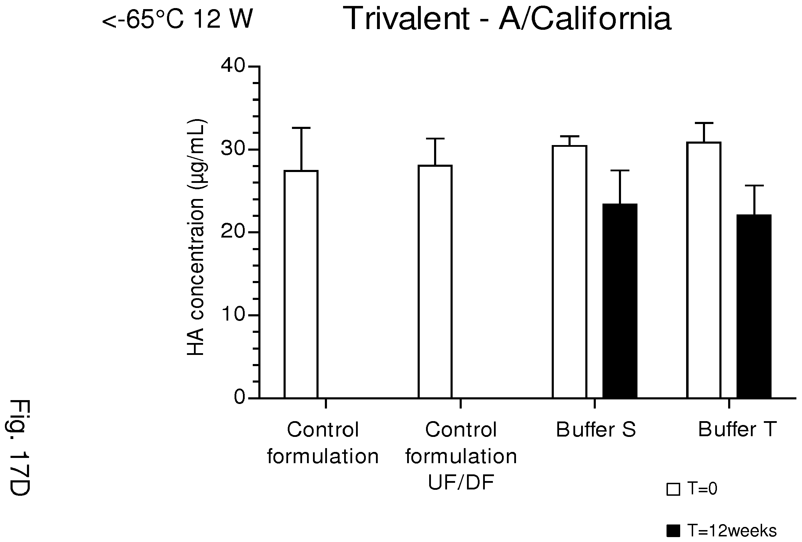

Trivalent virosomes were stored at <-65°C for 12 weeks and average diameter size and HA concentration were measured at t=0 and t=12 weeks (Fig. 17).

No significant variation was observed over time in terms of virosome size in the new formulations tested (Fig. 17A). The new formulations tested were capable of stabilizing the virosomes at <-65°C for 12 weeks, while a clear increase in virosome size was observed for the control samples (current formulation).

HA concentration was measured for each of the three strains included in the trivalent composition (Fig.17B, 17C and 17D) at t=0 and t=12 weeks storage at <-65°C. A clear improvement was observed for the two new formulations tested, compared to the controls (current formulation), especially for B/Brisbane and A/California. The HA concentration of these two strains drops dramatically after storage at <-65°C for 12 weeks in the control formulations, while is maintained essentially constant if the virosomes are formulated in the two new formulations tested.

Combining the data obtained as described above and after statistical analysis, a specific combination of variables has clearly demonstrated to provide the best stability for virosomes during storage at <-65°C. The composition that proved to be most robust for virosomal stability after storage at <-65°C was a buffered composition comprising 20 mM Na₂HPO₄/KH₂PO₄ at a pH of 7.5, 75 mM NaCl and further comprising 4% trehalose.

### REFERENCES

Freixeiro et al., "Study of the stability of proteoliposomes as vehicles for vaccines against Neisseria meningitidis based on recombinant porin complexes", Int J Pharm. 2013 Feb 25; 443(1-2):1-8. doi: 10.10 1 6/j.ijpharm.2012.12.046. Epub 2013 Jan 7.
Herzog et al., "Eleven years of Inflexal V-a virosomal adjuvanted influenza vaccine", Vaccine. 2009 Jul 16;27(33):4381-7. doi: 10.1016/j.vaccine.2009.05.029. Epub 2009 May 29.
Hincha et al. "Trehalose Increases Freeze-Thaw Damage in Liposomes Containing Chloroplast Glycolipids", Cryobiology. 1998 May;36(3):245-9.
Meunier F et al. "Liposomal amphotericin B(AmBisome): safety data from a phase II/III clinical trial." J Antimicrob Chemother. 1991;28 Suppl B:83-91.
Mishler et al, "Inflexal®V a trivalent virosome subunit influenza vaccine: production", Vaccine 20 (2002) B17-B23
Stegmann et al., "Functional reconstitution of influenza virus envelopes." EMBO J. 1987 Sep;6(9):2651-9.
Wood et al., "An improved single-radial-immunodiffusion technique for the assay of influenza haemagglutinin antigen: application for potency determinations of inactivated whole virus and subunit vaccines", J Biol Stand. 1977;5(3):237-47.

## Claims

1. A liquid composition comprising:
a) virosomes;
b) a KH₂PO₄/Na₂HPO₄ buffer, wherein the phosphate concentration is ranging between 15 mM and 30 mM;
c) a salt, wherein the salt concentration is higher than 60 mM;
d) trehalose, wherein the trehalose concentration is ranging between 2% w/w and 10% w/w, wherein said composition has a pH ranging between 6.5 and 8.

2. A composition according to claim 1, wherein said composition has a pH ranging between 7 and 7.8.

3. A composition according to claim 1, wherein said composition has a pH of 7.5; and comprises trehalose at a concentration ranging between 3% w/w and 5% w/w.

4. A composition according to any one of claims 1-3, further comprising a salt at a concentration ranging between 60 mM and 85 mM.

5. A composition according to claim 4, wherein said salt is NaCl.

6. A method of preserving a virosome which comprises preparing a composition according to any one of claims 1-5.

7. A method of preserving a virosome according to claim 6, further comprising storing said composition at a temperature ranging between 2°C and 8°C.

8. A method of preserving a virosome according to claim 6, further comprising storing said composition at a temperature ranging between -80°C and -65°C.

9. A method of preserving a virosome according to claim 6, further comprising storing said composition at a temperature ranging between -15°C and -30°C.

## Patentansprüche

1. Flüssige Zusammensetzung, umfassend:
a) Virosomen;
b) einen KH₂PO₄/Na₂HPO₄-Puffer, wobei die Phosphatkonzentration in einem Bereich zwischen 15 mM und 30 mM liegt;
c) ein Salz, wobei die Salzkonzentration höher als 60 mM ist;
d) Trehalose, wobei die Trehalosekonzentration in einem Bereich zwischen 2 Gew.-% und 10 Gew.-% liegt,
wobei die Zusammensetzung einen pH-Wert aufweist, der in einem Bereich zwischen 6,5 und 8 liegt.

2. Zusammensetzung nach Anspruch 1, wobei die Zusammensetzung einen pH-Wert aufweist, der in einem Bereich zwischen 7 und 7,8 liegt.

3. Zusammensetzung nach Anspruch 1, wobei die Zusammensetzung einen pH-Wert von 7,5 aufweist; und Trehalose in einer Konzentration umfasst, die in einem Bereich zwischen 3 Gew.-% und 5 Gew.-% liegt.

4. Zusammensetzung nach einem der Ansprüche 1-3, ferner umfassend ein Salz in einer Konzentration, die in einem Bereich zwischen 60 mM und 85 mM liegt.

5. Zusammensetzung nach Anspruch 4, wobei es sich bei dem Salz um NaCl handelt.

6. Verfahren zur Konservierung eines Virosoms, das Herstellen einer Zusammensetzung nach einem der Ansprüche 1-5 umfasst.

7. Verfahren zur Konservierung eines Virosoms nach Anspruch 6, ferner umfassend Lagern der Zusammensetzung bei einer Temperatur, die in einem Bereich zwischen 2°C und 8°C liegt.

8. Verfahren zur Konservierung eines Virosoms nach Anspruch 6, ferner umfassend Lagern der Zusammensetzung bei einer Temperatur, die in einem Bereich zwischen -80°C und -65°C liegt.

9. Verfahren zur Konservierung eines Virosoms nach Anspruch 6, ferner umfassend Lagern der Zusammensetzung bei einer Temperatur, die in einem Bereich zwischen -15°C und -30°C liegt.

## Revendications

1. Composition liquide comprenant :
a) des virosomes ;
b) un tampon KH₂PO₄/Na₂HPO₄, dans lequel la concentration de phosphate est dans la plage comprise entre 15 mM et 30 mM ;
c) un sel, la concentration de sel étant supérieure à 60 mM ;
d) du tréhalose, la concentration de tréhalose étant dans la plage comprise entre 2 % (m/m) et 10 % (m/m),
ladite composition ayant un pH dans la plage comprise entre 6,5 et 8.

2. Composition selon la revendication 1, ladite composition ayant un pH dans la plage comprise entre 7 et 7,8.

3. Composition selon la revendication 1, ladite composition ayant un pH de 7,5 ; et comprenant du tréhalose à une concentration dans la plage comprise entre 3 % (m/m) et 5 % m/m.

4. Composition selon l'une quelconque des revendications 1 à 3, comprenant en outre un sel à un concentration dans la plage comprise entre 60 mM et 85 mM.

5. Composition selon la revendication 4, dans laquelle ledit sel est NaCl.

6. Procédé de conservation d'un virosome qui comprend la préparation d'une composition selon l'une quelconque des revendications 1 à 5.

7. Procédé de conservation d'un virosome selon la revendication 6, comprenant en outre le stockage de ladite composition à une température dans la plage comprise entre 2 °C et 8 °C.

8. Procédé de conservation d'un virosome selon la revendication 6, comprenant en outre le stockage de ladite composition à une température dans la plage comprise entre -80 °C et -65 °C.

9. Procédé de conservation d'un virosome selon la revendication 6, comprenant en outre le stockage de ladite composition à une température dans la plage comprise entre -15 °C et -30 °C.
